# EUROPEAN PATENT APPLICATION

(11) **EP 4 495 105 A1**
(43) Date of publication of application: **22.01.2025**
(21) Application number: 23185723.6
(22) Date of filing: 17.07.2023
(51) Int. Cl.: C07D 251/04, C07D 317/18, C07D 295/18, C07C 67/00, C07C 69/00, C07C 231/00, C07C 233/00, C07C 253/00, C07C 255/00, C23F 11/00

(54) **3-(4-HYDROXYBUT-2-YNOXY)PROPANOIC ACID ESTER, AMIDE AND NITRILE DERIVATIVES AS CORROSION PROTECTION AGENTS**

(71) Applicant: BASF SE, 67056 Ludwigshafen am Rhein (DE); Albert-Ludwigs-Universität Freiburg, 79098 Freiburg (DE)
(72) Inventor: RUDOLF, Peter, 67056 Ludwigshafen am Rhein (DE); FUKUZUMI, Takeo, 67056 Ludwigshafen am Rhein (DE); GANSCHOW, Michael, 79104 Freiburg (DE); MERKENS, Kay, 79110 Freiburg (DE); RUEHE, Juergen, 79110 Freiburg (DE)
(74) Representative: BASF IP Association

(57) **Abstract**

The present invention relates to alkynyl ether compound of formula (I) 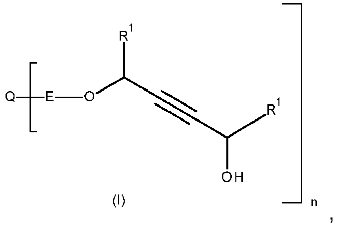 a process for the production of theses alkynyl ether compound of formula (I), mixtures of different alkynyl ether compounds of formula (I) in different solvents, the use of these alkynyl ether compounds of formula (I) or of their mixtures as corrosion protection agents or for the preparation of cyclic carbonytes with exocyclic vinyl groups of formula (IV)

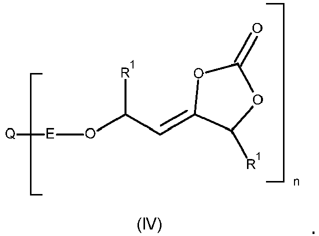

## Description

The present invention relates to alkynyl ether compound of formula (I) 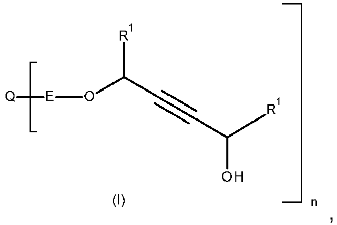 a process for the production of theses alkynyl ether compound of formula (I), mixtures of different alkynyl ether compounds of formula (I) in different solvents, the use of these alkynyl ether compounds of formula (I) or of their mixtures as corrosion protection agents or for the preparation of cyclic carbonates with exocyclic vinyl groups of formula (IV)

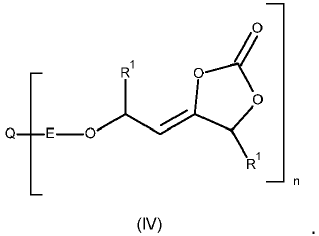

Exo-vinylene carbonates are valuable compounds, especially as monomers in polymer applications as described in WO 2011/157671 A1.

The Exo-vinylene carbonates mentioned in WO 2011/157671 were used as reactive diluent in epoxide resins and are all based on substituted propargyl alcohol. During the curing process of the epoxide the exo-vinylene carbonate reacts with the epoxide binder system and does not leave the epoxide resin. Thus, these special exo-vinylene carbonates based on propargyl alcohol described in WO 2011/157671 react as chain termination agents.

There is a high demand of exo-vinylene carbonates which acts as active monomers in a polymerization reaction. In order to be an active part in a polymerization reaction the exo-vinylene carbonates need at least one further functional group beside the exo-vinylene group. Exo-vinylene carbonates based on propargyl alcohols don't have a further functional group which can be used for the polymerization.

Therefore, the precursor compounds of the exo-vinylene carbonates need at least two functional groups, one to form the exo-vinylene group and another one for the polymerization reaction or to form a further exo-vinylene group.

Instead of propargyl alcohol dialcohols like 1 ,4-butynediole or 2,5-hexynediol might be better starting products for the precursor compounds of the exo-vinylene carbonates. But there are different risks mentioned in the prior art which seems to teach away from using dialcohols like 1,4-butyndiole or 2,5-hexyndiol.

US 4,487,781, WO 2020/113233, WO 2017/76998 A1 and Tv. Golobokova et al. in Zh. Org. Khim., 2013 vol 49, No. 1, p. 135-141 describe the oxo-Michael-addition reaction between propargyl alcohol with an acrylic acid ester for different steps in the synthesis of active components. The Oxo-Michael addition between one OH-group of 1,4-butynediol and/or 2,5-hexynediol with an α,β -unsaturated functional compound or the transesterification of the compound of formula (III) with an amine in order to get a precursor for an exo-vinylene carbonates with a further functional group is not described in these documents.

T.A. Favorskaya and S.A. Poroshina et al. describe in Zhurnal Organicbeskoi Khimii, Vol.6, No. 7, p 1416-1419, July 1970 the Oxo-Michael-Addition of 1,4-butynediol with methyl acrylate or acrylonitrile. They describe that they use two mols of methyl acrylate with one mol of 1,4-butynediol and isolate 24% of 2-(methoxycarbonyl) ethyl ether of butyne1,4-diol (the mono ester) via distillation and also receive 40% of the bis[2-(methoxycarbonyl)ethyl] ether of butyne-1,4-diol (the diester). The use of such mono esters for the preparation of exo-cyclic carbonates or as corrosion protection agents are not mentioned here.

Daniel Hennen et al. in ChemPhotoChem 2020, 4, 476-480 describe the oxa-Michael addition reaction between butanedioldiacrylate and propargyl alcohol. The received main product is BBP, but there are further by-products which are caused by transesterification, which is a big challenge during the oxa-Michael addition of alcohols to a diacrylate derivates as the purification is not easy. The selective oxa-Michael addition from an α,β- unsaturated functional compounds with 1,4-butynediol and/or 2,5-hexynediol to the alkynyl ether compounds of formula (I) as the main product without or limited amounts of transesterification side products is not disclosed. It is also not described that in an alternative way of production the alkynyl ether compounds of formula (I) can be received by transesterification of the compounds of formula (III) with an amine.

Karin Ratzenböck et al. also describes in Poly. Chem 11, 7476 the problem of transesterification during polymerisation of dialcohols with ethylacrylate.

WO 2019/034648 A1 describes the production of Exo vinylene carbonates by using propargylic alcohols with CO₂ in the presence of special transition metal catalyst, preferable Ag, with at least one bulky ligand, particular a phosphor ligand. The definition of "propargylic alcohols" mentioned in WO 2019/034648 is not restricted to 2-proyn-1-ol. It also means all compounds that comprises the prop-2-yn-1-ol group. Examples where 1,4-butynediol is reacted with a halogen aryl via a substitution reaction to the corresponding alkynyl ether compound are disclosed in WO 2019/034648. The Michael oxo addition products of 1,4-butynediol and/or 2,5-hexyndiol with an α,β -unsaturated compound to the alkynyl ether compound according to formula (I) or the transesterification of the compound of formula (III) with an amine to the inventive alkynyl ether compounds of formula (I) are not disclosed in WO 2019/034648.

M.S. Morad describes in Materials Chemistry and Physics 60 (1999) 188-195 that acetylenic compounds have been well known for decades as efficient inhibitors for metal corrosion in acid media, especially for iron and steel alloys. The simplest one among them is propargyl alcohol (2-propyn-1-ol or PA) which is used as an additive in acidizing of oil wells, in chemical cleaning and acid pickling of heat-transfer equipment, removing of scales in metallurgy, etc.

In Brubaker and Phipps; Corrosion Chemistry ACS Symposium Series; American Chemical Society: Washington, DC, 1979, page 288 ff it is mentioned that terminal propargyl alcohol group is considered the most active form. It is further noticed that substituents may enhance the corrosion protecting character of propargylic alcohol.

Nevertheless, low molecular acetylenic alcohols such as propargylic alcohol are challenged by their unfavorable tox-profile.

Therefore, it is an object of the present invention to provide alkynyl ether compounds including beside the propargyl group at least one further functional group which can be used as corrosion protection agents with better tox-profile or improved performance profile as propargylic alcohol as well as precursor compounds for exo-cyclic carbonates.

This problem will be solved with alkynyl ether compounds of formula (I) 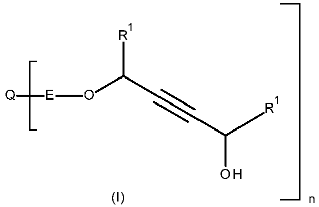 with
- n: is a natural number selected from the 1 to 10,
- R¹: is selected from the group of H and methyl,
- E: is selected from the group of -CH₂-CH₂-, -CH₂-CH₂-C(O)-, and 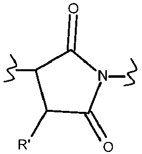 with R' is selected from the group of H and a C₁-C₈ alkyl group, wherein the carbon atom of the terminal -CH₂- or the -CH- group next to the -CHR'-group of E is bonded to the -O- atom of formula (I)
- Q: is dependent on n and E
for n=1 and E= -CH₂-CH₂-
   Q is selected from the group of -CN and substituted or unsubstituted 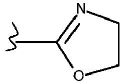 (2-oxa-zolin) group,
for n=1 and E= -CH₂-CH₂-C(O)-
   Q is selected from the group of -O-Y, -NR²-Y and substituted or unsubstituted 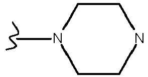 (piperazine),
for n=1 and E= 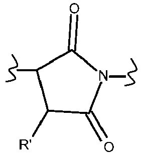 Q is selected from the group of substituted or unsubstituted C₆-C₄₀ aryl groups,
for n=2 and E= -CH₂-CH₂-
   Q is selected from the group of a divalent bonded sulfone (-S(O)₂-) group, a divalent substituted or unsubstituted 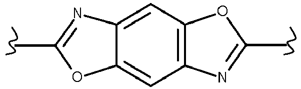 group and a divalent substituted or unsubstituted 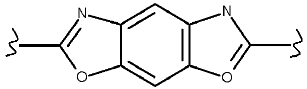 (benzobisoxazol-2,6-diyl) group,
for n=2 and E= -CH₂-CH₂-C(O)-
   Q is a divalent -A- group,
for n=2 and E= 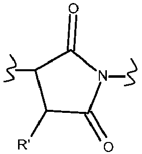 Q is selected from the group of divalently bonded substituted or unsubstituted C₄-C₁₀ alkylen groups, divalently bonded substituted or unsubstituted phenyl, divalently bonded substituted or unsubstituted C₁₃-C₄₀ arylalkylenaryl groups and divalently bonded substituted or unsubstituted C₈-C₄₀ alkylenarylalkylen groups, for n=3 and E= -CH₂-CH₂-C(O)-
   Q is selected from the group of a substituted or unsubstituted 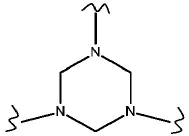 (1,3,5-tria-zinan) group, 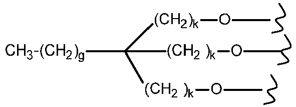 group, 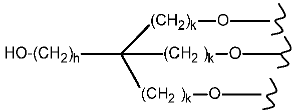 group and 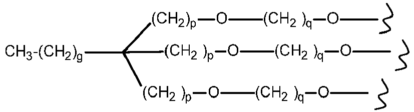 group wherein
   g is a natural number selected from 1 to 33,
   k is a natural number selected from 1 to 12,
   h is a natural number selected from 1 to 15,
   p is a natural number selected from 1 to 3 and
   q is a natural number selected from 1 to 5,
for n≥4 and E= -CH₂-CH₂-C(O)-
   Q is selected from the group of 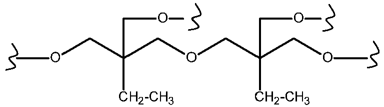 and 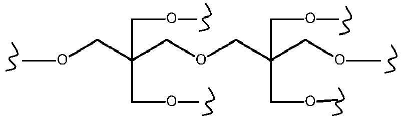
and
- with R²: selected from the group of H, substituted or unsubstituted C₁-C₄₀ alkyl groups, substituted or unsubstituted C₃-C₄₀ cycloalkyl groups, substituted or unsubstituted saturated or unsaturated heterocyclic groups containing 2 to 20 carbon atoms and one or more heteroatoms selected from the group of N, O, and S as ring members, substituted or unsubstituted C₆-C₄₀ aryl groups, substituted or unsubstituted C₂-C₄₀-alkenyl groups, C₂-C₄₀ alkynyl groups, substituted or unsubstituted C₄-C₄₀ alkylencycloalkyl groups and substituted or unsubstituted C₁-C₄- alkylen groups to be a part of a substituted or unsubstituted saturated or unsaturated heterocyclic group that is built up between R² of the -NR²- group and Y to form 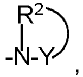
- with Y: is selected from the group of substituted or unsubstituted C₁-C₄₀ alkyl groups, substituted or unsubstituted saturated or unsaturated C₃-C₄₀ cycloalkyl groups, substituted or unsubstituted C₂-C₄₀ (poly)ether groups, substituted or unsubstituted saturated or unsaturated heterocyclic groups containing 2 to 20 carbon atoms and one or more heteroatoms selected from the group of N, O, and S as ring members, substituted or unsubstituted saturated or unsaturated alkylenheterocyclic groups containing 3 to 20 carbon atoms and one or more heteroatoms selected from the group of N, O, and S as ring members, substituted or unsubstituted C₆-C₄₀ aryl groups, substituted or unsubstituted C₂-C₄₀ alkenyl groups, substituted or unsubstituted C₂-C₄₀ alkynyl groups, substituted or unsubstituted saturated or unsaturated C₃-C₄₀ alkylencycloalkyl groups, substituted or unsubstituted C₇-C₄₀ alkylenaryl groups, substituted or unsubstituted α,β- unsaturated carbonyl groups containing C₃-C₄₀ atoms, substituted or unsubstituted C₂-C₅ keto groups
and substituted or unsubstituted C₁-C₄ alkylen group which is part of a substituted or unsubstituted saturated heterocyclic group that is built up between R² of the -NR²- group and Y to form 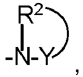
- with -A-: is one of the following bridging groups: -X¹-Z-X²- or 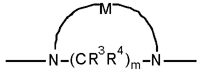 wherein X¹, X² are independently selected from -O-, and-NR²- and
Z is selected from the group of substituted or unsubstituted C₁-C₄₀ alkylen groups, divalently substituted or unsubstituted C₂-C₄₀ (poly)ether groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded substituted or unsubstituted alkylenheterocyclic-alkylen groups containing 4 to 20 carbon atoms and one or more heteroatoms selected from the group of N, O, and S as ring members, divalently bonded substituted or unsubstituted C₆-C₄₀ aryl groups, divalently bonded substituted or unsubstituted C₇-C₄₀ cycloalkylalkylencylcoalkyl groups, divalently bonded substituted or unsubstituted saturated or unsaturated C₅-C₄₀ alkylencycloalkylalkylen groups, divalently bonded substituted or unsubstituted C₁₃-C₄₀ arylalkylenaryl groups, divalently bonded substituted or unsubstituted C₈-C₄₀ alkylenarylalkylen groups, 2-butyne-yl group and divalently bonded substituted or unsubstituted C₁₇-C₃₀ alkylen-bisphenol A -alkylen groups and
R³ and R⁴ are independently selected from the group of H, substituted or unsubstituted C₁-C₄₀ alkyl groups,
m is a natural number selected from the group of 1, 2 or 3 and
M is selected from the group of substituted or unsubstituted C₁-C₃ alkylen groups, divalently bonded -NR⁵-CR³R⁴-groups, divalently bonded -NR⁵-NR⁶- groups, divalently bonded -NR⁵-CR³R⁴-CR³R⁴- groups, divalently bonded-O-CR³R⁴-CR³R⁴- groups, divalently bonded -S-CR³R⁴-CR³R⁴- groups, divalently bonded CR³R⁴-O-CR³R⁴ groups, divalently bonded -CR³R⁴-S-CR³R⁴- groups and divalently bonded -CR³R⁴-NR⁵-CR³R⁴- groups
with R⁵ and R⁶ are independently selected from the group of H, substituted or unsubstituted C₁-C₄₀ alkyl groups, C₆-C₄₀ aryl groups, C₃-C₄₀ cycloalkyl groups, and-C(O)CH=CH₂ group
wherein the alkynyl ether compounds of formula (I) with
n =1, R¹ = H, E = -CH₂-CH₂- and Q= -CN or with
n = 1, R¹ = H, E =-CH₂-CH₂-C(O)- and Q = -O-Y with Y = methyl or ethyl are disclaimed.

The inventive alkynyl ether compound will be advantageous, wherein E = -CH₂-CH₂- , n is selected from the group of 1 and 2 and Q is selected from the group of -CN, with C₁-C₄ alkyl groups or C₁-C₄ alkylhydroxy groups substituted 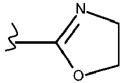 (2-oxazolin) groups, a divalent bonded sulfone (-S(O)₂-) group, a divalent substituted or unsubstituted 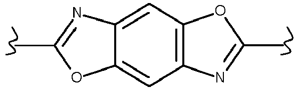 group and a divalent substituted or unsubstituted 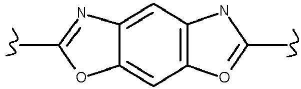 (benzobisoxazol-2,6-diyl) group.

The inventive alkynyl ether compounds will be advantageous, wherein E = 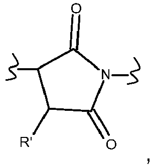 n is selected from the group of 1 and 2 and Q is selected from the group of phenyl, substituted or unsubstituted divalently bonded C₆-C₄₀ aryl groups, divalently bonded substituted or unsubstituted C₁₃-C₄₀ arylalkylenaryl groups, divalently bonded substituted or unsubstituted C₈-C₄₀ alkylenarylalkylen groups.

The inventive alkynyl ether compounds will be advantageous, wherein E = 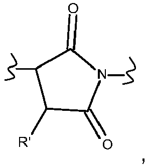 n is selected from the group of 1 and 2 and Q is selected from the group of phenyl, 1,4-phenylen, 1,3-phenylen, 4-Methyl-1,3-phenylen, 2,2,4-trimethyl-1,6-hexylen, 1,3-Benzenedimethylene (meta -CH₂-Ph-CH₂-), bis(1,4-phenylen)methane (para -CH₂-Ph-CH₂-) and 3,3'-Diethyl-5,5'-dimethyl-4,4'-diphenylenmethane.

The inventive alkynyl ether compounds will be advantageous, wherein E = -CH₂-CH₂-C(O)-, n is selected from the group of 1 and 2 and Q is selected from the group of -O-Y and -O-Z-O-.

The inventive alkynyl ether compounds, wherein E = -CH₂-CH₂-C(O)-, n is selected from the group of 1 and 2 and Q is selected from the group of -O-Y and -O-Z-O, wherein Y is selected from the group of substituted or unsubstituted C₁-C₄₀ alkyl groups, substituted or unsubstituted saturated or unsaturated C₃-C₄₀ cycloalkyl groups, substituted or unsubstituted saturated alkylenheterocyclic groups containing 3 to 20 carbon atoms and one or more heteroatoms selected from the group of N, O, and S as ring members and substituted C₃-C₄₀ unsaturated alkylencycloalkyl groups and
Z which is selected from the group of substituted or unsubstituted C₁-C₄₀ alkylen groups, divalently bonded substituted or unsubstituted C₃-C₄₀ cycloalkyl groups, divalently bonded substituted or unsubstituted saturated heterocyclic groups containing 2 to 20 carbon atoms and one or more heteroatoms selected from the group of N, O, and S as ring members, divalently bonded saturated substituted or unsubstituted C₅-C₄₀ alkylencycloalkylalkylen groups and divalently bonded substituted or unsubstituted C₁₇-C₃₀ alkylen-bisphenol A - alkylen groups.

The inventive alkynyl ether compounds according will be advantageous, wherein E = -CH₂-CH₂-C(O)-, n is selected from the group of 1 and 2 and Q is selected from the group of -NR²-Y, substituted or unsubstituted 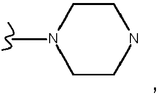 -NR²-Z-NR²-, -NR²-Z-O- and 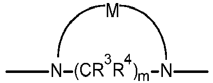 group, wherein R², Y, Z, R³, R⁴, M and n are as defined above.

The inventive alkynyl ether compounds will be advantageous, wherein E = -CH₂-CH₂-C(O)-, n is selected from the group of 1 and 2 and Q is selected from the group of -NR²-Y, substituted or unsubstituted 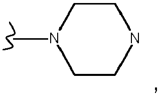 -NR²-Z-NR²-, -NR²-Z-O- and 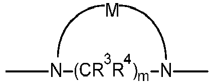 group with
- R²: is selected from the group of H and substituted or unsubstituted C₁-C₄₀ alkyl groups,
- Y: is selected from the group of substituted or unsubstituted C₁-C₄₀ alkyl groups and substituted or unsubstituted C₆-C₄₀ aryl groups, the substituted 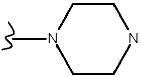 group is a 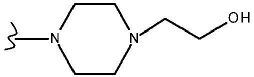 (4-(2-hydroxy-ethyl)piperazin-1-yl group,
- Z: is selected from the group of substituted or unsubstituted C₁-C₄₀ alkylen groups, divalently bonded substituted or unsubstituted C₆-C₄₀ aryl groups and divalently bonded substituted or unsubstituted alkylenheterocyclicalkylen groups containing 4 to 20 carbon atoms and one or more heteroatoms selected from the group of N, O, and S as ring members,
- M: is -CH₂-CH₂-,
- R³ and R⁴: are H and
- m: is 2.

The inventive alkynyl ether compounds will be advantageous, wherein E = -CH₂-CH₂-C(O)-, n is 3 and Q is selected from the group of unsubstituted 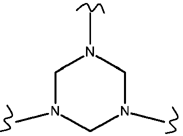 (1,3,5-triazinan) group, 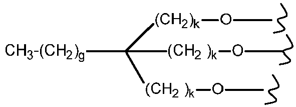 group, 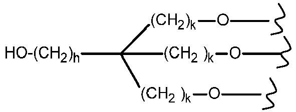 group and 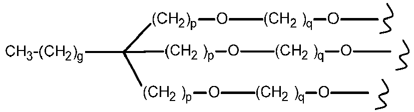 group wherein
- g: is a natural number selected from 1 to 33,
- k: is a 1,
- h: is a natural number selected from 1 to 15,
- p: is a 1 and
- q: is a natural number selected from 1 to 5.

The inventive alkynyl ether compounds will be advantageous, wherein E = -CH₂-CH₂-C(O)-, n ≥ 4 and Q is selected from the group of 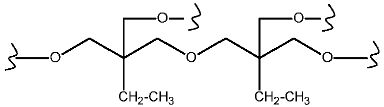 and

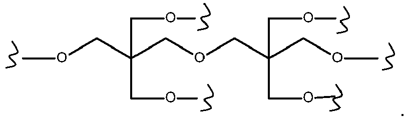

A further embodiment of the invention is a process for the preparation of the inventive alkynyl ether compound of formula (I) 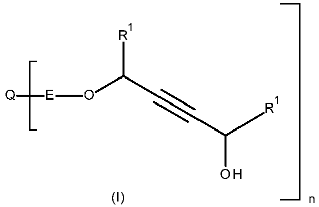 comprising the reaction of an α,β- unsaturated functional compound selected from the group of compounds of formula (II) 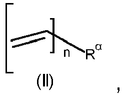 maleinimide-N-Q-group 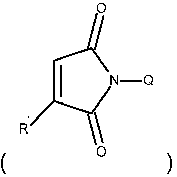 and a maleinimide-N-Q-N-maleinimide group 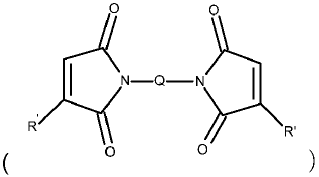 with Q as defined above for the case where n is 1 or 2 and E is 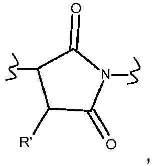 with 1,4,-butynediol and/or 2,5-hexynediol, wherein for n = 1 R^{α} is selected from the group of -CN, 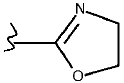 (2-oxazolin), -C(O)-O-Y and -C(O)-NR²-Y, for n = 2 R^{α} is selected from the group of -C(O)-A-C(O)-, -S(O)₂- and 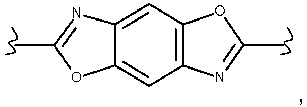 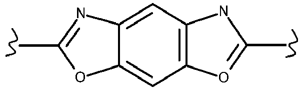 (benzobis oxazol-2,6-diyl), for n = 3 R^{α} is selected from the group of 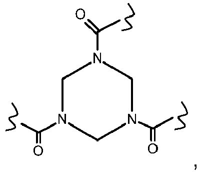 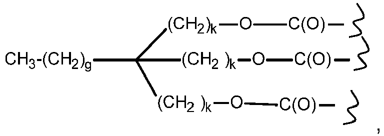 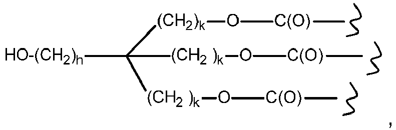 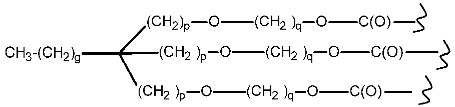 wherein g, k, h, p and q are defined as above, for n ≥ 4 R^{α} is selected from the group of 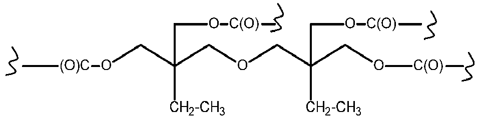 and 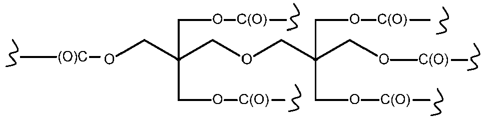 with R', Y, R² and A as defined above.

A further embodiment of the invention is an alternative process for the preparation of the inventive alkynyl ether compound of formula (I) where E= -CH₂-CH₂-C(O)- and Q is selected from the group of NR²-Y and -X¹-Z-X²- wherein the compounds where X¹ and X² are both O are disclaimed 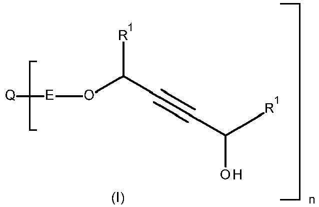 comprising the following steps:
a) reacting an α,β- unsaturated carbonyl compound of formula (Ila) 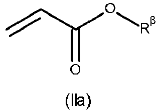
   with R^{β} selected from the group of C₃-C₁₀ alkyl groups,
   with 1,4,-butynediol and/or 2,5-hexynediol,
   wherein the resulting product of step a), which is the ester of formula (III) or a mixture of two E/Z-isomers of the ester of formula (III), 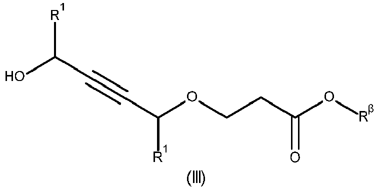
b) reacts in a further step b) with a compound selected from the group of an amine, diamine, triamine and substituted or unsubstituted ethanolamine,
   wherein R¹, Y, R², Z, X¹, X² and n are as defined above.

The alternative inventive process will be advantageously, wherein the resulting product of step a) reacts with a compound selected from the group of HNR²-Y, H₂N-Z-NH₂, 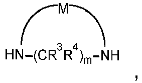 N-substituted piperazine, substituted or unsubstituted 1,3,5-triazinane and H₂N-Z-OH,
wherein Y, R², Z, M, R³, R⁴ and m are as defined above.

The inventive process as well as the alternative inventive process will be advantageous, wherein the molar ratio between 1,4-butynediol and/or 2,5-hexynediol and α,β- unsaturated functional compound of formula (II) or α,β- unsaturated carbonyl compound of formula (IIa) is in the range from 1:1 to 10:1.

The inventive process as well as the alternative inventive process will be advantageous, wherein the temperature during the reaction of the α,β- unsaturated functional compound of formula (II) or α,β- unsaturated carbonyl compound of formula (IIa) with 1,4-butynediol and/or 2,5-hexynediol will be in the range from 25°C to 120°C, at normal pressure and a catalyst is used.

The inventive process as the alternative inventive process will be advantageous, wherein an alkaline catalyst is used during the reaction of the α,β- unsaturated functional compound of formula (II) or α,β- unsaturated carbonyl compound of formula (IIa) with 1,4-butynediol and/or 2,5-hexynediol.

The inventive process will be advantageous, wherein no solvent is used during the reaction of the α,β- unsaturated functional compound of formula (II) or α,β- unsaturated carbonyl compound of formula (Ila) with 1,4-butynediol and/or 2,5-hexynediol.

The inventive process will be advantageous, wherein the molar ratio between 1,4-butynediol and/or 2,5-hexynediol and α,β- unsaturated functional compound of formula (II) or α,β- unsaturated carbonyl compound of formula (IIa) is in the range from 3:1 to 10:1.

The inventive process will be advantageous, wherein a polar organic solvent is used during the reaction of the α,β- unsaturated functional compound of formula (II) or α,β- unsaturated carbonyl compound of formula (IIa) with 1,4-butynediol and/or 2,5-hexynediol.

The inventive process will be advantageous, wherein an aqueous solvent is used during the reaction of the α,β- unsaturated functional compound of formula (II) or α,β- unsaturated carbonyl compound of formula (Ila) with 1,4-butynediol and/or 2,5-hexynediol.

The inventive process will be advantageous, wherein the aqueous solvent is water only and the molar ration between 1,4-butynediol and/or 2,5-hexynediol and α,β- unsaturated functional compound of formula (II) or α,β- unsaturated carbonyl compound of formula (IIa) is in the range from 1:1 to 3:1.

The inventive process will be advantageous, wherein the α,β- unsaturated functional compound of formula (II) or α,β- unsaturated carbonyl compound of formula (IIa) is dissolved in a polar organic solvent and 1,4-butynediol and/or 2,5-hexynediol are dissolved in water.

The alternative inventive process will be advantageous, wherein the α,β- unsaturated functional compound of formula (II) or α,β- unsaturated carbonyl compound of formula (IIa) is either dissolved in an organic solvent or no solvent.

A further embodiment of the invention is an aqueous mixture obtained by the inventive process wherein the aqueous solvent is water only.

A further embodiment of the invention is a mixture obtained by the inventive process comprising at least two alkynyl ether compounds of formula (I), wherein in one alkynyl ether compound of formula (I) R¹ is H and in the other alkynyl ether compound of formula (I) R¹ is methyl.

A further embodiment of the invention is the use of the inventive alkynyl ether compounds for the preparation of cyclic carbonates with exocyclic vinylidene groups of formula (IV) 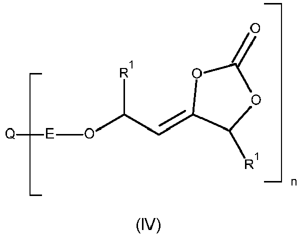 wherein R¹, E, Q and n are as defined above.

A further embodiment of the invention is the use of the inventive alkynyl ether compounds of formula (I) as corrosion protection agent.

A further embodiment of this invention is the use of the inventive mixture for the preparation of a mixture of cyclic carbonates with exocyclic vinylidene groups of formula (IV) where in some cyclic carbonates with exocyclic vinylidene groups R¹ is H and in the others R¹ is methyl.

A further embodiment of this invention is the use of the inventive aqueous mixture as corrosion protection agent.

The term "alkyl" group as used in the present application comprises linear or singly or multiply branched saturated hydrocarbons. Preferably are linear, singly or multiply branched saturated C₁-C₂₂-alkyl groups, more preferably are singly or multiply branched saturated C₁-C₁₀ alkyl, much more preferably are C₁-C₈ alkyl groups selected from the group of methyl, ethyl, n-propyl, isopropyl, n-butyl, sec-butyl, tert.-butyl, 1,1-dimethylethyl n-pentyl, 2-methylbutyl, 2,2-dimethyl propyl, n-hexyl, 2-methylpentyl, 3-methylpentyl, 2,2,-dimethylbutyl, 2,3-dimethylbutyl, n-heptan, 2-methylhexyl, 3-methylhexyl, 2,2-dimethylpentyl, 2,3-dimethylpentyl, 3,3-dimethylpentyl, 2,4-dimethylpentyl, 3-ethylpentyl, 2,2,3 trimethylbutyl, n-octyl, 2-methylheptyl, 3-methylheptyl, 4-methylheptyl, 2,2-dimethylhexyl, 2,3-dimethylhexyl, 2,4-dimethylhexyl, 2,5-dimethylhexyl, 3,3,-dimethylhexyl, 3,4-dimethylhexyl, 3-ethylhexyl, 2,2,3-trimethylpentyl, 2,2,4-trimethylpentyl, 2,3,3-trimethylpentyl, 2,3,4-trimethylpentyl, 3-ethyl-2-methylpentyl, 3-ethyl-3-methylbutyl, 2,2,3,3-tetramethylbutyl, most preferred are methyl, ethyl, n-propyl, isopropyl, n-butyl, sec-butyl, tert.-butyl, n-pentyl, 2-methylbutyl, 2,2-dimethyl propyl, n-hexyl, 2-methylpentyl, 3-methylpentyl, 2,2,-dimethylbutyl, 2,3-dimethylbutyl. Most preferred C₁-C₅ alkyl groups are selected from the group of methyl, ethyl, n-propyl, isopropyl, n-butyl, sec-butyl, tert.-butyl, 1,1-dimethylethyl, n-pentyl, 2-methylbutyl, 2,2-dimethylpropyl. Much more preferably are C₁-C₅ alkyl groups selected from the group of methyl, ethyl, n-propyl, isopropyl, n-butyl, sec-butyl, tert-butyl, 1,1-dimethylethyl and 2,2-dimethylpropyl.

The term "alkylen" group as used in the present application does not mean alkenes having double bonds but comprises a C₁-C₂₀, preferably C₁- C₁₀, more preferably C₁-C₆ divalent bonded linear or with C₁-C₄ alkyl groups substituted saturated hydrocarbons having free valencies on adjacent or terminal carbon atoms. Preferably the alkylen group is selected from the group of methylene (-CH₂-), ethylene (-CH₂-CH₂-), n-propylene (-CH₂-CH₂-CH₂-), isopropylene (-CH(CH₃)-CH₂-), 2,2,-dimethylpropylene (-CH₂-C(CH₃)₂-CH₂-), n-butylene (-CH₂-CH₂-CH₂-CH₂-) n-pentylene (-CH₂-CH₂-CH₂-CH₂-CH₂) and n-hexylene (-CH₂-CH₂-CH₂-CH₂-CH₂-CH₂-).

The term "alkylhydroxide" group as used in the present application comprises C₁-C₂₂ alkyl groups where at least one H atom of the alkyl chain is substituted by one or more OH groups. Preferably the alkylhydroxide is selected from the group of methylhydroxide, ethylhydroxide, n-propylhydroxide, 2-methylpropylhydroxid, 2,2-dimethylpropylhydroxide, 2-hydroxypropyl, 1-dodecanylhydroxid (lauryl), 1-hexandecanylhydroxid (cetyl), 1-octadecanylhydroxid (stearyl) and 1-docosanylhydroxid (behenyl). More preferably are methyl hydroxide, ethylhydroxide, 2-methylpropylhydroxid, 2,2-dimethylpropylhydroxide, and 2-hydroxypropyl.

The term "cycloalkyl" group as used in the present application comprises C₃-C₄₀ containing monocyclic or polycyclic aliphatic hydrocarbon groups wherein the term "polycyclic" comprises two or more cycloalkyl rings which are independently to each other connected by normal C-C bond, by a spiro-bond (two cycloaliphatic rings share one sp³ hybridized C-atom), by sharing at least one C-C bond, which is part of two cycloaliphatic ring systems (condensed ring system) or by sharing at least two C-atoms in at least two cycloaliphatic ring systems where a C₁-C₃-alkylen group is in between (bridged ring systems). Preferably the monocyclic aliphatic groups are selected from the group of cyclopropanyl, cyclobutanyl, cyclopentanyl, cyclohexanyl, cycloheptanyl and cyclooctanyl. More preferred monocyclic aliphatic groups are selected from the group of cyclobutanyl, cyclopentenyl and cyclohexanyl. The preferred polycyclic aliphatic groups are selected from the group of bicyclopentanyl, bicyclohexanyl, spiropentanyl, spiroheptanyl, spiroundecanyl, spiro [4.5] decanyl, dispiro-[4.2.2.5.2]pentadecanyl, decalinyl, bicyclo[2.2.1]heptyl, bicyclo[3.1.1]heptyl, tricyclo[5.2.1.0]decanyl and Trimethylbicyclo[2.2.1]hept-2-yl-. More preferred polycyclic aliphatic groups are selected from the group of bicyclopentanyl, bicyclo[2.2.1]heptyl and tricyclo[5.2.1.0]decanyl

The term "alkylencycloalkyl" group used in the present application comprises a C₁ - C₁₅ containing alkylen group wherein at least one H is substituted by a mono- or polycyclic C₃ - C₃₉ containing cycloalkyl group wherein the sum of the carbon atoms in the alkylencycloalkyl group is not higher than 40. Preferably are the alkylencycloalky groups wherein the alkylen group is selected from the group of methylen, ethylen, n-propylen, isopropylen, n-butylen, sec-butylen and tert.-butylen group and the cycloalkyl group is selected from C₃- C₁₂ cycloalkyl groups. More preferably the alkylencylcoalkyl group is selected from the group of methylencyclopropan, methylencyclobutan, methylencyclopentan, methylencyclohexan, methylenbicyclopentan, methylenbicyclohexan, methylennorbornan, ethylencyclopentan, ethylencyclohexan, ethylenbicyclopentan, ethylenbicyclohexan, ethylennorbornan, n-propylencyclohexan, isopropylencyclohexan, isopropylenbicyclopentan, isopropylenbicyclohexan, isopropylennorbornan, n-butylencyclopentan, n-butylencyclohexan, n-butylenbicyclopentan, n-butylenbicyclohexan, n-butylennorbornan, sec-, butylenbicyclopentan, sec-butylenbicyclohexan, sec-butylenspiropentan, sec-butylennorbornan, tert-butylencyclopentan, tert-butylencyclohexan, tert-butylenbicyclopentan, tert-butylenbicyclohexan and tert-butylennorbornan. Most preferably the alkylencycloalkyl group is selected from the group of methylencyclopentan, methylencyclohexan, methylenbicyclopentan and isopropylencyclohexan.

The term "alkylencycloalkylalkylen" group used in the present application comprises a C₅-C₄₀ containing group which is divalent and comprises a cycloalkyl group in the center and two identical or different alkylen groups at each side. The alkylencycloalkylalkylen group is a bridging group wherein the alkylen groups at each side are bonded either to two different or identical groups or which are bonded to the same or to different atoms of one group. The alkylen groups are independently selected from the group of methylen, ethylen, propylene and isopropylen. The cycloalkyl group is selected from the group of 1,2-cyclopropan, 1,2-, 1,3-, 1,4-cyclobutan, 1,2-, 1,3-clyopentan, 1,2-, 1,3-, 1,4-cyclohexan and 4,8- tricyclo[5.2.1.0^{2,6}]decan. The numbers in front of the cycloalkyl groups symbolize the C-atoms of the cycloalkylring which are bonded to the alkylen groups. Preferably alkylencycloalkylalkylen groups are selected from the group of 1,3-dimethylencyclopentan, 1,3 dimethylencyclohexan, 1,2-dimethylencyclohexan, 1,4-dimethylencyclohexan 1,3-diethylencyclopentan, 1,4-diethylencyclohexan, 1,3-dipropylencyclopentan, 1,4-dipropylencyclohexan and 4,8-bis(methylene)tricyclo[5.2.1.0^{2,6}]decan. More preferably the alkylencycloalkylen groups are selected from the group of 1,3 dimethylencyclohexan, 1,2-dimethylencyclohexan and 1,4-dimethylencyclohexan.

The term "cycloalkylalkylencycloalkyl" group used in the present application comprising a C₇-C₄₀ atoms containing group which is divalent and comprises an alkylen chain in the center, wherein the alkylen chain is selected from the group of methylene, ethylene, propylene, isopropylene and at least two mono-, two poly- or one mono- and one polycycloalkyl groups are bonded at each side of the alkylen chain. The cycloalkyl groups at each side of the alkylen chain are the same or different. The mono- and/or polycycloalkyl groups at each side of the alkylen chain will be the connection to a further group at each side of the mono- and/or polycycloalkyl group which is either identical or different. Preferably monocycloalkyl groups are independently from each other selected from the group of cyclopropanyl, 1,2- and 1,3-cyclobutanyl, 1,2- and 1,3-cyclopentanyl and 1,2-, 1,3- and 1,4-cylohexanyl. More preferably monocycloalkyl groupts are independently from each other selected from the group of 1,3-cyclobutanyl, 1,3-cyclopentanyl, 1,2- and 1,4-cylohexanyl. The numbers in front of the cycloalkyl groups symbolize the C-atoms of the cycloalkylring which are bonded on the one side to the alkylen group and on the other side to the further group. Preferably polycycloalkyl groups are independently from each other selected from the group of tricyclo[5.2.1.0^{2,6}]decanyl, 2-norbornyl, bornyl, isobornyl, 2-decahydronaphthyl, tetracyclo-[4.4.0.1^{2,5}.1^{7,10}]docecyl. Preferably cycloalkylalkylencycloalkyl groups are selected from the group of dicyclopropanylmethan, 1,2- and 1,3-dicyclobutanylmethan, 1,2- and 1,3-dicyclopentanylmethan, 1,2-, 1,3- and 1,4-dicyclohexanylmethan, dicyclopropanylethan, 1,2- and 1,3-dicyclobutanylethan, 1,2- and 1,3-dicyclopentanylethan, 1,2-, 1,3- and 1,4-dicyclohexanylethan, dicyclopropanylpropan, 1,2- and 1,3-dicyclobutanylpropan, 1,2- and 1,3-dicyclopentanylpropan, 1,2-, 1,3- and 1,4-dicyclohexanylpropan, 1,2- and 1,3-dicyclobutanylisopropan, 1,2- and 1,3-dicyclopentanylisopropan, 1,2-, 1,3- and 1,4-dicyclohexanylisopropan, cyclopropanyl-1,2- or 1,3-cyclobutanylmethan, cyclopropanyl-1,2- or 1,3-cyclopentanylmethan, cyclopropanyl-1,2- or 1,3- or 1,4-cyclohexanylmethan, 1,2- or 1,3-cyclobutanyl-1,2- or 1,3-cyclopentanylmethan, 1,2- or 1,3-cyclobutanyl-1,2 or 1,3 or 1,4-cyclohexanylmethan, 1,2- or 1,3-cyclopentanyl-1,2 or 1,3 or 1,4-cyclohexanylmethan, cyclopropanyl-1,2 or 1,3-cyclobutanylethan, cyclopropanyl-1,2 or 1,3-cyclopentanylethan, cyclopropanyl-1,2 or 1,3 or 1,4-cyclohexanylethan, 1,2 or 1,3-cyclobutanyl-1,2 or 1,3-cyclopentaylethan, 1,2 or 1,3-cyclobutanyl-1,2 or 1,3 or 1,4-cyclohexanylethan, 1,2- or 1,3-cyclopentanyl-1,2 or 1,3- or 1,4-cyclohexanylethan, cyclopropanyl-1,2- or 1,3-cyclobutanylpropan, cyclopropanyl-1,2- or 1,3-cyclopentanylpropan, cyclopropanyl-1,2- or 1,3- or 1,4-cyclohexanylpropan, 1,2- or 1,3-cyclobutanyl-1,2 or 1,2-cyclopentaylpropan, 1,2- or 1,3-cyclobutanyl-1,2 or 1,3 or 1,4-cyclohexanylpropan and 1,2 or 1,3-cyclopentanyl-1,2 or 1,3 or 1,4-cyclohexanylpropan. More preferably cycloalkylalkylencycloalkyl groups are selected from the group of 1,4-dicyclohexanylmethan, 1,3-dicyclopentanylethan and 1,4-dicyclohexanylisopropan.

The term "unsaturated" cycloalkyl, alkylencycloalkyl, alkylencycloalkylalkylen, cycloalkylalkylencycloalkyl or alkylenheterocyclicalkylen group used in the present application comprises C₃-C₄₀ cycloalkyl, a C₄-C₄₀ alkylencycloalkyl, a C₅-C₄₀ alkylencycloalkylalkylen, a C₇-C₄₀ cycloalkylalkylencycloalkyl or a C₄-C₄₀ alkylenheterocyclicalkylen groups that include at least one C-C double bond inside of one or more of the rings, wherein no double bond is located at the bridge head of a bridged bi- or multicycloalkyl ring system. Preferably unsaturated monocyclic cycloalkyl groups are selected from the group of cyclopentenyl, cyclohexenyl, cyclooctenyl and cyclododecenyl. Preferably unsaturated polycyclic cycloalkyl group are selected from the group of dihydrodicyclopentadienyl and norbornenyl. Preferably unsaturated monocyclic alkylencycloalkyl groups are selected from the group of (cyclohex-3-en-1-yl)methylene and (4-methyl-1-cyclohexene)isopropylene. Preferably unsaturated polycyclic alkylencycloalkyl groups are selected from the group of Bicyclo [2.2.1]hept-5-ene-2-methylene, 2-Methyl-bicyclo [2.2.1]hept-5-ene-2-methylene, 3a,4,5,6,7,7a-Hexahydro-4,7-methano-1*H*-indene-5-methylene and 3a,4,5,6,7,7a-Hexahydro-4,7-methano-1*H*-indene-6-methylene. Preferably unsaturated monocyclic alkylencyloalkylalkylen groups are selected from the group of 4-Cyclopentene-1,3-dimethylene and 2-Cyclohexene-1,4-dimethylene. Preferably unsaturated monocyclic alkylenheterocyclicalkylen groups is 2,5-furandimethylene.

The term "aryl" group used in the present application comprises C₆-C₄₀ containing mono- or polycyclic aromatic hydrocarbon groups wherein the term "polycyclic aromatic" comprises two or more rings wherein the whole ringsystem is aromatic and in which the rings are connected by a normal C-C-bond or by sharing at least one C-C bond, which is part of two rings (condensed ring systems). Preferably mono- or polycyclic aromatic hydrocarbon groups are selected from the group of phenyl, biphenyl, naphthyl, anthracenyl, phenanthrenyl, indenyl, as-indacenyl, fluorenyl. Most preferred are phenyl and naphthyl.

The term "alkylenaryl" group used in the present application comprises a C₇ to C₄₀ containing group that is build up by an alkylen chain that is bonded to a mono- or polycyclic aryl group. Preferably are the alkylenaryl groups wherein the alkylen chain is selected from the group of methylene, ethylene, n-propylene, iso-propylene, n- butylene, sec-butylene and tert.-butylene group and the aryl group is selected from C₆ to C₁₂ aryl groups. More preferably the alkylenaryl group is selected from the group of benzyl, methylenbiphenyl, methylennaphthalenyl, methylenanthracenyl, methylenphenanthrenyl, methylenindenyl,methylene-as-indacenyl, methylenfluorenyl, ethylenphenyl, ethylenbiphenyl, ethylennaphthalenyl, ethylenanthracenyl, ethylenphenanthrenyl, ethylenindenyl, ethylene-as-indacenyl, ethylenfluorenyl, n-propylenphenyl, n-propylenbiphenyl, n-propylenanthracenyl, n-propylenphenanthrenyl, n-propylenindenyl, n-propylene-as-indacenyl, n-propylenfluorenyl, iso-propylenphenyl, iso-propylenbiphenyl, iso-propylenantracenyl, isopropylenphenantrenyl, iso-propylenindenyl, iso-propylene-as-indacenyl, iso-propylenfluorenyl, n-butylenphenyl, n-butylenbicyclophenyl, n-butylennaphthalenyl, n-butylenanthracenyl, n-butylenphenanthrenyl, n-butylenindenyl, n-butylene-as-indacenyl, n-butylenfluorenyl, sec-butylenphenyl, sec-butylenbiphenyl, sec-butylennaphthalenyl, sec-butylenanthracenyl, sec-butylenphenanthrenyl, sec-butylenindenyl, sec-butyleneas-indacenyl, sec-butylenfluorenyl, tert.-butylenphenyl, tert.-butylenbicyclophenyl, tert.-butylennaphthalenyl, tert.-butylenanthracenyl, tert.-butylenphenanthrenyl, tert.-butylenindenyl, tert.-butylene-as-indacenyl, tert.-butylenfluorenyl. Most preferred alkylenaryl groups are selected from benzyl, ethylenphenyl, iso-propylenphenyl and tert.-butylenphenyl.

The term "arylalkylenaryl" group used in the present application comprising a C₁₃-C₄₀ atoms containing group which is divalent and comprises at least two aryl groups which are connected via an C₁-C₁₀ containing alkylen chain. The connection to the arylalkylenaryl group will be build to each of the two aryl groups. Preferably the alkylen chain, which is in the center of the two aryl groups, is selected from the group of methylene, ethylene, propylene and isopropylene. At each side of the center alkylen chain an aryl group is connected. These aryl groups can be identical or different. As each aryl group is further connect to a further group, which also can be the same or different, at each side of the arylalkylenaryl group, each aryl group has two connection carbon atoms which are identified here by the number of the carbon atom of the aryl group that build up the connection to the center alkylen group as well as the number of the carbon atom that build up the connection to the further group on the other side of the aryl group. Preferably the aryl groups are independently selected from the group of 1,2-, 1,3- and 1,4-phenyl, 1,2-, 1,3-, 1,4-, 1,5-,1,6-, 1,7-, 1,8-, 2,3-, 2,4-, 2,5-, 2,7- 2,8-, 3,4-, 3,5-, 3,6-, 3,7- and 3,8-naphthyl, 1,2-, 1,3-, 1,4-, 1,5-, 1,6-, 1,7-, 1,8-, 1,9-, 1,10-, 2,3- 2,4-, 2,5-, 2,6-, 2,7-, 2,8-, 2,9-, 2,10-, 3,4-, 3,5-, 3,6-, 2,7- 3,8-, 3,9- and 3,10-anthracenyl. Preferably arylalkylenaryl groups are selected from the group of bis(1,4-phenyl)methane, bis(2,5-naphthyl)methan, bis(2,7-anthracenyl)methan, 1,1-bis(1,4-phenyl)ethane, 1,1-bis(2,5-naphthyl)ethan, 1,1-bis(2,7-anthracenyl)ethan, 1,2-bis(1,4-phenyl)ethane, 1,2-bis(2,5-naphthyl)ethan, 1,2-bis(2,7-anthracenyl)ethan, 1,3-bis(1,4-phenyl)propan, 1,3-bis(2,5-naphthyl)propan, 1,3-bis(2,7-anthracenyl)propan, 1,2-bis(1,4-phenyl)propan, 1,2-bis(2,5-naphthyl)propan, 1,2-bis(2,7-anthracenyl)propan, 1,1-bis(1,4-phenyl)propan, 1,1-bis(2,5-naphthyl)propan, 1,1-bis(2,7-anthracenyl)propan 2,2-bis(1,4-phenyl)propan, bis(2,5-naphthyl)isopropan, bis(2,7-anthracenyl)isopropan, (1,4-phenyl),(2,5-naphthyl)methane, (1,4-phenyl),(2,7-anthracenyl)methan, (2,5-napthyl),(2,7-antracenyl)methan, 1-(1,4phenyl),2-(2,5-naphthyl)ethane, 1-(1,4-phenyl),2-(2,7anthracenyl)ethan, 1-(2,5-napthyl),2-(2,7-antracenyl)ethan, 1-(1,4phenyl),1-(2,5-naphthyl)ethane, 1-(1,4-phenyl),1-(2,7-anthracenyl)ethan, 1-(2,5-napthyl),1-(2,7-antracenyl)ethan, 1-(1,4-phenyl),3-(2,5-naphthyl)propan, 1-(1,4-phenyl),3-(2,7-anthracenyl)propan, 1-(2,5-napthyl),3-(2,7-antracenyl)propan, 1-(1,4-phenyl),2-(2,5-naphthyl)propan, 1-(1,4-phenyl),2-(2,7-anthracenyl)propan, 1-(2,5-napthyl),2-(2,7-antracenyl)propan, 1-(1,4-phenyl),1-(2,5-naphthyl)propan, 1-(1,4-phenyl),1-(2,7-anthracenyl)propan, 1-(2,5-napthyl),1-(2,7-antracenyl)propan, (1,4-phenyl)-(2,5-naphthyl)isopropan, (1,4-phenyl)-(2,7-anthracenyl)isopropan, (2,5-napthyl)-(2,7-antracenyl)isopropan, Most preferred arylalkyenaryl groups are selected from the group of 2,2-bis(1,4-phenyl)propan, bis(1,4-phenyl)methane.

The term "alkylenarylalkylen group" used in the present application comprising C₈-C₄₀ atoms containing group which is divalent and comprises at least two alkylen groups which are connected via one aryl group. The aryl group is in the center of the alkylenarylalkylen group and has two different carbon atoms in the ring to which each alkylen group is bonded. The two carbon atoms of the center aryl group are identified by the numbers of the two carbon atoms in front of the aryl group to which the same or different alkylen groups are bonded. The alkylen groups are independently of each other selected from the group of methylene, ethylene, propylene, and iso-propylene. The center aryl group is selected from the group of 1,2-phenyl, 1,3-phenyl, 1,4-phenyl. Preferred alkylenarylalkylen group is selected from the group of 1,2-dimethylenphenyl (-CH₂-1,2-phenyl-CH₂-) and 1,4-dimethylenphenyl (-CH₂-1,4-phenyl-CH₂-) group.

The term "heterocyclic group" used in the present application comprises saturated or unsaturated heterocyclic groups that contains 2 to 20 carbon atoms and one or more heteroatoms selected from the group of N, O and S as ring members. These heterocyclic groups can be divided in heterocycloalkyl groups and unsaturated heterocyclic groups. The term "unsaturated heterocyclic group" used in the present application comprises partially unsaturated heterocyclic groups as well as heteroaromatic groups. All of these heterocyclic groups can be a monoheterocyclic as well as a polyheterocyclic groups wherein at least in one cyclus at least one heteroatom is included. Preferred heterocycloalkyl groups are selected from the group of ethylenoxidyl (oxiranyl), ethylensulfidyl (thiiranyl), ethyleniminyl (aziridinyl), trimethylenoxidyle (oxetanyl), trimethylensulfidyl (thietanyl), 1,3-propyleniminyl (azetidinyl), tetrahydrofuranyl (oxolanyl), tetrahydrothiophenyl (thiolanyl), pyrrolidyl (azolidinyl), tetrahydropyranyl (oxanyl), tetrahydrothiopyranyl (thianyl), piperidinyl (azinanyl), hexamethylenoxidyl (oxepanyl), hexamethylensulfidyl (thiepanyl), tetrahydro-1,4,-oxazinyl (morpholin), tetrahydro-1,3,-oxazinyl, tetrahydro-1,2,-oxazinyl hexamethyleniminyl (azepanyl), , 1,3-dioxolanyl, 1,3-dithiolanyl, 1,3-oxathiolanyl, 1,3-oxaazolidnyl, 1,3-azathiolanyl, 1,3-dioxanyl, 1,4-dioxanyl, 1,3,5-trioxanyl, 1,3-dithianyl, 1,4-dithianyl, 1,3,5-trithianyl, 1,3-diazinanyl, 1,4-diazinanyl, 1,2,3-trianzinanyl, 1,2,4-triazinanyl, 1,3,5-triazinanyl (hexahydro-1,3,5-triazinyl), 1,2-oxazinanyl, 1,3-oxazinanyl, 1,4-oxazinanyl, octahydro-1-benzofuranyl, octahydro-2-benzofuranyl, hexahydro-furo[3,2-b]furanyl. More preferred heterocycloalkyl groups are selected from the group of ethylenoxidyl (oxiranyl), trimethylenoxidyle (oxetanyl), tetrahydrofuranyl (oxolanyl), piperidinyl (azinanyl), 1,3-dioxolanyl, 1,3-oxaazolidnyl, 1,3-dioxanyl, 1,3,5-trioxanyl, 1,3,5-triazinanyl (hexyhydro-1,3,5-triazinyl) and hexahydro-furo[3,2-b]furanyl. Preferred heteroaromatic or heteroaryl groups, which means the same, for the inventive functionalized cyclic carbonates of formula (I) as well as in the alkynyl ether compounds of formula (II) are selected from the group of pyrrolyl (2-pyrrolyl), pyrazolyl (3-pyrazolyl), imidazolyl (1-imi_dazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2- quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4- isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydrobenzo[b]furanyl (2-(2,3-di_hydro-benzo[b]furanyl), 3-(2,3-dihydro-benzo[b]furanyl), 4-(2,3-dihydro-benzo[b]furanyl), 5-(2,3-dihydrobenzo-[b]fura_nyl), 6-(2,3-dihydro-benzo-[b]furanyl), 7-(2,3-dihydro-benzo[b]furanyl)), benzo[b]thiophenyl (2-benzo[b]thiophenyl, 3- benzo[b]thiophenyl, 4-benzo[b]thiophenyl, 5-benzo[b]thiophenyl, 6-benzo[b]thiophenyl, 7-benzo[b]thiophenyl), 2,3-di_hydro-benzo[b]thiophenyl (2-(2,3-dihydro-benzo[b]thiophenyl), 3-(2,3-dihydrobenzo[b]thiophenyl), 4-(2,3-dihydroben_zo[b]thiophenyl), 5-(2,3-dihydro-benzo[b]thiophenyl), 6-(2,3-dihydro-benzo[b]thiophenyl), 7-(2,3-dihydro-benzo[b]thi_ophenyl)), 4,5,6,7-tetrahydrobenzo[b]thiophenyl (2-(4,5,6,7-tetrahydro-benzo[b]thiophenyl), 3-(4,5,6,7-tetrahydroben_zo[b]thiophenyl), 4-(4,5,6,7-tetrahydro-benzo[b]thiophenyl), 5-(4,5,6,7-tetrahydrobenzo[b]thiophenyl), 6-(4,5,6,7-tet_rahydro-benzo[b]thiophenyl), 7-(4,5,6,7-tetrahydrobenzo[b]thiophenyl)), thieno[2,3-b]thiophenyl, 4,5,6,7-tetrahydro_thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tet_rahydro-thieno[2,3-c]pyridyl), 7-(4,5,6,7-tetrahydro-thieno[2,3-c]pyridyl)), indolyl (1-indolyl, 2-indolyl, 3-ihdolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7- isoindolyl), 1,3-dihydro-isoindolyl (1-(1,3-dihydro-isoindolyl), 2-(1,3-dihydroisoindolyl), 3-(1,3-dihydro-isoindolyl), 4-(1,3- dihydro-isoindolyl), 5-(1,3-dihydro-isoindolyl), 6-(1,3-dihydro-isoindolyl), 7-(1,3-dihydro-isoindolyl)), indazole (1-inda_zolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxa_zolyl, 2-benzoxazolyl), benzothiazolyl (1-benzothiazolyl, 2-benzothiazolyl, 4-benzothiazolyl, 5-benzothiazolyl, 6-benzo_thiazolyl, 7-benzothiazolyl), benzo-[1,2,5]oxadiazolyl, (4-benzo[1,2,5]oxadiazole, 5-benzo[1,2,5]oxadiazole), carbazolyl (1-carbazolyl, 2-carbazolyl, 3-carbazolyl, 4-carbazolyl), piperidinyl (2-piperidinyl, 3-piperidinyl, 4-piperidinyl), pyrrolidinyl (1-pyrrolidinyl, 2-pyrrolidinyl, 3- pyrrolidinyl), benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,5-a]pyridinyl, pyrrolo[1,2-b]pyridazinyl, imidazo[1,2-c]pyrimidinyl, quinolinyl, isoquinolinyl, cinnolinyl, quinazolinyl, quinoxalinyl, phthalazinyl, pyrido[3,2-d]pyrimidinyl, pyrido[4,3-d]pyrimidinyl , pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "alkylenheterocyclic" group used in the present application comprises a C₁-C₄₀ atoms containing group that comprises a C₁-C₂₀ alkylen chain wherein one H- atom is substituted by an hetereocyclic group. The alkylen chain is preferably selected from the group of methylene, ethylene, n-propylene, isopropylen, n-butylen, sec-butylen and tert.- butylene. The hetereocyclic group is preferably selected from C₄-C₃₉ heterocycloalkyl groups and aromatic heterocyclic groups. Preferably the alkylenheterocyclic group is selected from the group of 4-Methylen-1,3-dioxolan, 3-methylen-oxetan, 2-methylene furan and 2-methylene tetrahydrofuran.

The term "alkylenheterocyclicalkylen" group used in the present application comprises either an aromatic heterocyclic or a saturated heterocycloalkyl group in the center which comprises two directly at the C₂-C₃₈ containing hetereocyclic group bonded C₁-C₁₀ containing substituted or unsubstituted alkylen groups. These two C₁-C₁₀ alkylen groups are independently selected from the group of methylene, ethylene, n-propylene, iso-propylen, n-butylene, sec-butylene and tert.-butylene groups which can be substituted or unsubstituted. Both C₁-C₁₀ containing alkylen groups are the divalent bondages to further groups which can be identical or different. Preferably heterocyclic groups are selected from the group of tetrahydrofuran, tetrahydrothiophene, pyrrolidine, tetrahydropyran, tetrahydrothiopyran, piperidin, 1,3- or 1,4 - dioxolan, 1,3- or 1,4 -dithian,1,3- or 1,4-piperazin, furan, thiophen, pyrrol and pyridine. Most preferably alkylenheterocyclicalkylen groups are selected from the group of 2,5-dimethylentetrahydrofuran and 2,5-dimethylenfuran.

The term "alkenyl" group used in the present application comprises C₂-C₄₀ linear hydrocarbons having one or more C-C double bonds can be cumulated, alternating or statistic. Preferably alkenyl groups are selected from the group of ethenyl (vinyl), allyl, 1-propenyl, buten-1-yl, buten-2-yl, buta-1,3- dienyl, buta-1,2-dienyl, butatrienyl, penten-1-yl, penten-2-yl, penta-1,2-dienyl, penta-1,3-dienyl, penta-1,4-dienyl. More preferred alkenyl groups are selected from the group of ethenyl (vinyl), allyl and 1-propenyl.

The term "alkynyl" group used in the present application comprises C₂ to C₄₀ linear hydrocarbons having one or more C-C triple bonds can be alternating or statistic. Preferably alkynyl groups are selected from the group of ethynyl, propynyl, butyn-1-yl, butyn-2-yl, buta-1,3-diynyl, pentyn-1-yl, pentyn-2-yl, hexyn-3-yl. More preferably alkynyl groups are selected from the group of ethynyl, propynyl, butyn-2-yl and hexyn-3-yl.

The term "(poly)ether" group used in the present application comprises mono- and polyether groups. Monoether groups are those groups where only one -O- atom is connected to two C atoms of different or identical organyl groups (C-groups) at each side. The C-groups at each side of these monoether groups are independently selected from the group of C₁-C₃₉ containing alkylen, alkyl, mono- or divalent aryl, mono- or divalent cycloalkyl, unsaturated mono- or divalent cycloalkyl and mono- or divalent heterocyclic groups. Polyether groups are those groups where at least three C-groups or more are interrupted after each C-group by one -O-atom. If all the C-groups in the mono- or polyether groups are identical, these groups are symmetric mono- or polyether groups. If the C-groups are different, than these are unsymmetric mono- or polyether groups. (Poly)ether groups are bonded with one C-atom of one C-group to a further group, which is either the -O- or -N- atom of the -O-Y-group or of the -NR²-Y group or the (poly)ether group is the bridging group Z between X¹ and X² of the -X¹-Z-X²- group, where two C-groups of the (poly)ether group are bonded each with one C-atom to the -O- and/or N- atom of the X¹ and X² group. The C-groups for the polyether groups are the same as those for the monoether groups. Preferably (poly)ether groups are those with 2 to 40 C- atoms overall included C-groups. Preferably symmetric monoether groups are selected from the group of methoxymethylen, methoxyoligo(methyloxy) (CH₃-[O-CH₂]_{w}-), ethoxyethylen, n-propoxy-n-propylen, n-butoxy-n-butylen and phenoxy-ethylen. Preferably symmetric polyether groups are selected from the group of (-CH₂-CH₂-O-)_{w}-CH₂-CH₂-, (-CH₂-CH₂-CH₂-O-)_{w}-CH₂-CH₂-CH₂-, (-C(CH₃)H-CH₂-O-)_{w}-C(CH₃)H-CH₂-, (-CH₂-C(CH₃)H-O-)_{w}-CH₂-C(CH₃)H-, (-C(CH₃)₂-CH₂-O-)_{w}-C(CH₃)₂-CH₂-, (-CH₂-CH(CH₃)-O-)_{w}-CH(CH₃)-CH₂-, (-CH₂-CH₂-CH₂-CH₂-O-)_{w}-CH₂-CH₂-CH₂-CH₂-, (-C(CH₃)H-CH₂-CH₂-O-)_{w}-C(CH₃)H-CH₂-CH₂-, (-CH₂-C(CH₃)H-CH₂-O-)_{w}-CH₂-C(CH₃)H-CH₂-, (-CH₂-CH₂-C(CH₃)H-O-)_{w}-CH₂-CH₂-C(CH₃)H-CH₂-, (-C(CH₃)₂-CH₂-O-)_{w}-C(CH₃)₂-CH₂-, (-C(CH₃)H-C(CH₃)H-O-)_{w}-C(CH₃)H-C(CH₃)H-and (-CH₂-C(CH₃)₂-O-)_{w}-CH₂-C(CH₃)₂- with w which can be any natural number from 2 to 38.

Preferably unsymmetric monoether groups are selected from the group of methoxyethylen, methoxy-n-propylen, methyoxy-isoproylen, methoxy-n-butylen, methoxy-isobutylen, methoxy-tert.-butylen, ethoxymethylen, ethoxy-n-propylen, ethoxy-isopropylen, ethoxybutylen, ethoxy-isobutylen, ethoxy-tert.-butylen, n-propoxymethylen, n-propoxyethylen, n-propoxy-isoproylen, n-propoxy-n-butylen, n-propoxy-isobutylen, n-propoxy-tert.-butylen, isopropoxymethylen, isopropoxyethylen, isopropoxy-n-propylen, isopropoxy-n-butylen, isopropoxy-isobutylen, isoproxy-tert.-butylen, cyclopentanoxyethylen cyclohexanoxyethylen, phenoxyethylen, dicyclopentanyloxyethylen and dicyclopentenyloxyethylen. More preferred unsymmentric monoether groups are selected from the group of methoxyethylen, ethoxybutylen, cyclopentaoxyethylen, cyclohexanoxyethylen, phenoxyethylen, dicyclopentanyloxyethylen and dicyclopentenyloxyethylen.

Preferably unsymmetric (poly)ether groups are selected from the group of C₁-C₃₉-alkylen-O-C₁-C₃₉-alkylen, C₁-C₃₇-alkylen-O-C₃-C₃₉-cycloalkyl, C₁-C₃₇-alkylen-O-C₃-C₃₉-unsaturated cycloalkyl, C₁-C₃₄-alkylen-O-C₆-C₃₉-aryl, C₁-C₃₈-alkylen-O-C₂-C₂₀-heterocyclic group, C₃-C₃₇-cycloalkyl-O-C₃-C₃₇-cycloalkyl, C₃-C₃₇-cycloalkyl-O-C₃-C₃₇-unsaturated cycloalkyl, C₃-C₃₄-cycloalkyl-O-C₆-C₃₇-aryl, C₃-C₃₈-cycloalkyl-O-C₂-C₂₀-heterocyclic group, C₃-C₃₇unsaturated cycloalkyl-O-C₃-C₃₇-unsaturated cycloalkyl, C₃-C₃₄-unsaturated cycloalkyl-O-C₆-C₃₇ aryl, C₃-C₃₈-unsaturated cycloalkyl-O-C₂-C₂₀-heterocyclic group, C₆-C₃₄-aryl-O-C₆-C₃₄-aryl, C₆-C₃₈-aryl-O-C₂-C₂₀-heterocyclic group and C₂-C₁₈-heterocyclic group-O-C₂-C₁₈-heterocyclic group, wherein the number of C-atoms in both C-containing groups of each side of the -O- group are different, if the C-containing groups belong to the same kind of C-containing groups. More preferably unsymmetric (poly)ether groups are selected from the group of C₁-C₃₉-alkylen-O-C₁-C₃₉-alkylen, C₁-C₃₇-alkylen-O-C₃-C₃₉-cycloalkyl, C₁-C₃₉-alkylen-O-C₁-C₃₉-alkylen and C₁-C₃₇-alkylen-O-C₃-C₃₉-cycloalkyl, wherein the number of C-atoms in both C-containing groups of each side of the -O- group are different, if the C-containing groups belong to the same kind of C-containing groups.

The term "alkyloxy" group used in the present application is a special kind of mono- or polyether group and means an alkylen group that ends with an -O-atom. This -O-atom is, in contrast to the (poly)ether groups, bonded to a further group, which is the C-atom of the carbonyl group (-C(O)-) of E in formula (I). With the -C-atom of its alkylen group the alkyoxy group is bonded either to the -O-atom of a further (same or different) alkyloxy group, to a divalent bonded -O-atom or to a C-atom of the hydrocarbon chain that is substituted by the alkyloxy group. Preferably alkyloxy groups are C₁-C₂₀- alkyloxy groups. More preferably are those alkyloxy groups that are selected from the group of oxymethylen (-CH₂-O-), oxyethylen (-CH₂-CH₂-O-), oxy-n-propylen (-CH₂-CH₂-CH₂-O-), oxy-iso-propylen (CH₂-CH(CH₃)-O-), oxy-n-butylen (-CH₂-CH₂-CH₂-CH₂-O-), oxy-n-pentylen (-CH₂-CH₂-CH₂-CH₂-CH₂-O-), 2-(2-ethoxyethoxy)ethanol (ethyldiglycerol) (CH₃-CH₂-O-[CH₂-CH₂-O-]₂-), 2-(2-butoxyethoxy)ethanol (butyldiglycerol) (CH₃-CH₂-CH₂-CH₂-O-[CH₂-CH₂-O-]₂-) and 2-(2-Methoxyoligoethoxy)ethanol (methoxyoligoethylenglycol) (CH₃-O-[CH₂-CH₂-O-]_{y}-) with y= a natural number starting from 3 to 10). Most preferably are oxymethylen (-CH₂-O-), oxyethylen (-CH₂-CH₂-O-), oxy-n-propylen (-CH₂-CH₂-CH₂-O-), oxy-iso-propylen (CH₂-CH(CH₃)-O-), ethyldiglycerol (CH₃-CH₂-O-[CH₂-CH₂-O-]₂-), butyldiglycerol (CH₃-CH₂-CH₂-CH₂-O-[CH₂-CH₂-O-]₂-) and methoxyoligoalkyleneglycol (CH₃-O-[CH₂-CH₂-O-]_{y}-) with y= a natural number selected from the group of 3, 4, 5 and 6 .

The term "keto" group used in the present application comprises at least a C₃-C₁₉ comprising carbon chain that is interrupted by at least one or more >C=O (carbonyl) groups that are not terminal (-C-C(O)-C-) wherein the carbon chains on both sides of the carbonyl group are independently selected from the group of substituted or unsubstituted C₁-C₈ alkyl, substituted or unsubstituted C₆-C₁₀ cycloalkyl and substituted or unsubstituted C₆- C₈ aryl groups. Preferably keto groups are selected from the group of methylketon (CH₃-C(O)-C-), ethylketon (CH₃-CH₂-C(O)-C-), propylketon (CH₃-CH₂-CH₂-C(O)-C-), butylketon (CH₃-CH₂-CH₂-CH₂-CH₂-C(O)-C-), pentylketon (CH₃-CH₂-CH₂-CH₂-CH₂-C(O)-C-), (CH₃-C(O)-CH₂-C(O)-) to be derived from acetoacetoxyethylmethacrylate (AAEM), (CH₃-C(O)-CH₂-C(CH₃)₂-) to be derived from diacetone acrylamide (DAAM), (H₃C-C(O)-CH₂-CH₂-C(O)-) to be derived from levulinic acid and CH₃-C(O)-CH₂-CH₂- to be derived from methylvinyl ketone. More preferably keto groups are selected from the group of methylketon (CH₃-C(O)-C-), ethylketon (CH₃-CH₂-C(O)-C-), (CH₃-C(O)-CH₂-C(O)-) to be derived from acetoacetoxyethylmethacrylate (AAEM), (CH₃-C(O)-CH₂-C(CH₃)₂-) to be derived from diacetone acrylamide (DAAM), (H₃C-C(O)-CH₂-CH₂-C(O)-) to be derived from levulinic acid and CH₃-C(O)-CH₂-CH₂- to be derived from methylvinyl ketone.

The term "α,β- unsaturated carbonyl" groups used in the present application comprises C₃-C₄₀ atoms and at least one C-C double bond in directly neighborhood to a >C=O (carbonyl group) which can be an aldehyde carbonyl group, an keto carbonyl group, an acid carbonyl group, ester carbonyl, an imide carbonyl group or an amide carbonyl group. Preferably α,β-unsaturated carbonyl group are selected from the group of α,β- unsaturated ester carbonyl (H₂C=CH-C(O)-O∼), (H₂C=C(CH₃)-C(O)-O∼), (H₃C-HC=CH₂-C(O)-O∼), HOOC-HC=CH₂-C(O)-O∼, HOOC-C(CH₃)=CH-C(O)-O∼, HOOC-CH=C(CH₃)-C(O)-O∼, HOOC-CH₂-C(=CH₂)-C(O)-O∼, α,β- unsaturated imide carbonyl group (maleimide or H₂C=CH-C(O)-NR-C(O)-C∼) and α,β- unsaturated amid carbonyl groups (H₂C=CH-C(O)-N<, H₂C=C(CH₃)-C(O)-N<). More preferably is the α,β- unsaturated carbonyl group selected from the group of (H₂C=CH-C(O)-O∼), (H₂C=C(CH₃)-C(O)-O∼), (H₃C-HC=CH₂-C(O)-O∼), HOOC-HC=CH₂-C(O)-O∼, HOOC-C(CH₃)=CH-C(O)-O∼, HOOC-CH=C(CH₃)-C(O)-O∼, HOOC-CH=C(CH₃)-C(O)-O∼, HOOC-CH₂-C(=CH₂)-C(O)-O∼ and H₂C=CH-C(O)-N<.

The term "substituted or unsubstituted" used in present application means that the described group can carry instead of one or more than one H-atoms, no, one or more substituents which are independently selected from the group of -OH, -CN, -C(O)N, -F, C₁-C₆ alkyl groups, C₁-C₆-alkylhydroxide groups, C₃-C₆ cycloalkyl groups, C₄-C₁₀ alkylencycloalkyl groups, C₃-C₆ unsaturated cycloalkyl groups, C₅-C₆ aryl groups, C₇- C₁₀ alkylenaryl groups, heterocyclic groups containing 3 to 5 carbon atoms and one or more heteroatoms selected from the group of N, O and S as ring members, (poly)ether groups containing C₂-C₂₀ carbon atoms, C₁-C₁₈ alkyloxy group, keto groups containing C₂-C₅ carbon atoms, α,β-unsaturated carbonyl groups containing C₃-C₃₀ carbon atoms, C₂-C₆ alkenyl groups, C₂-C₆ alkynyl groups and -NR⁸R⁹ groups, wherein R⁸ and R⁹ are independently selected from the group of H, C₁-C₆ alkyl groups, C₃-C₆- cycloalkyl groups, and C₅ -C₆ aryl groups wherein the sum of all carbon atoms in the main chain or main cyclic group and in the chain or cyclic group that are used as substituent will not be higher than the highest carbon atoms amount that is mentioned in front of the main chain or main cyclic group.

The term "divalent" used in the present application comprising the ability of a group to be bonded to two different or same groups. These divalent groups were used as bridging groups which connect two parts of the molecule which are different or the same. These connections will be built at both ends of the bridging group and result to a longer chain or connect two other parts of the molecule via ring closure. The atoms of the divalent group that will be used for the connection to the other parts of the molecule are preferably independently selected from the group of -CH₂-, two >CH-groups of a C₃-C₆ cycloalkyl group, two >CH-groups of a C₃-C₆ heterocycloalkyl group, a -CH₂-group of each alkyl group of a C₈-C₄₀... alkylenarylalkylen group and a -CH- group of each aryl group of a C₁₃-C₄₀ arylalkylenaryl groups.

Preferably OH substituted heterocyloalkyl group is 5-Hydroxy-1,3-dioxan.

Preferably alkylhydroxid substituted heterocycloalkyl groups are selected from the group of (2-Methyloltretrahydrofuran (tetrahydrofurfuryl) and 4-Methylol-1,3-dioxolan.

Preferabyl alky and alkyloxy substituted heterocycloalkyl groups are selected from the group of 5-Ethyl-5-methylol- 1,3-dioxan, 3-Ethyl-3-methyloloxetan and 2,2-Dimethyl-4-methylol-1,3-dioxolan (solketal).

Preferably alky substituted heterocycloalkyl groups are selected from the group of 1,2,2,6,6,-Pentamethylpiperid-3-yl and 1,2,2,6,6,-Pentamethylpiperid-4-yl.

Preferably alkyl and alkenyl substituted unsaturated cycloalkyl groups is 4-(isoprop-2-yl)-1-methylcyclohexene

Preferably alkyl substituted cycloalkyl groups are selected from the group of 3,3,5-Trimethylcyclohexanyl, 4-tert.-Butylcyclohexanyl and 1,1,3,3-Tetramethylcyclobutan-2,4-diyl.

Preferably -NR⁸R⁹ substituted alkyl group is dimethylaminopropyl.

Preferably -NCO substituted alkyl group is f 2-isocyanatoethyl.

Preferably -CN substituted alkyl groups is cyanatoethyl.

Preferably -F substituted alkyl groups are selected from the group of pentadecyfluorooctyl and trifluormethyl.

Preferably unsaturated cycloalkyl substituted alkylen groups is 2[4-Methylcylcohex-3-en-1-yl]propane-2-yl.

In the inventive alkynyl ether compounds of formula (I) R¹ is either H or CH₃ and n is a natural number from 1 to 10, the E-group is selected from the group of -CH₂-CH₂-, -CH₂-CH₂-C(O)- and 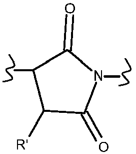 with R' which is selected from the group of H and a C₁-C₈ alkyl group,
wherein the carbon atom of the terminal -CH₂- or the -CH- group next to the -CHR'- group of E is bonded to the -O- of formula (I), Q dependents on E and n and divides the inventive alkynyl ether compounds of formula (I) into inventive alkynyl ether compounds with special functional groups selected from the group of monovalent nitrile, monovalent oxazoline, divalent sulfone, divalent benzobisoxazole, mono-, di- or multivalent esters and mono-, di or trivalent amides wherein the mono- or diester as special embodiment of the ester group is a bisphenol A- compound and the mono- or divalent amides as special embodiment of the amide group is a mono- or divalent maleimides wherein the alkynyl ether compounds of formula (I) selected from the group of Methyl-3-[(4-hydroxy-2-butyn-1-yl)oxy]propanoate, Ethyl-3-[(4-hydroxy-2-butyn-1-yl)oxy]propanoate and 3-[(4-Hydroxy-2-butyn-1-yl)oxy]propanenitrile are excluded.

Preferred are the inventive alkynyl ether compounds of formula (I) wherein E is selected from the group of -CH₂-CH₂- and -CH₂-CH₂-C(O)-, so that the inventive alkynyl ether compounds of formula (I) carry special functional groups selected from the group of monovalent nitrile, mono-, di- or multivalent esters, mono-, di- or trivalent amides, monovalent oxazoline, divalent sulfones and divalent benzobisoxazole, wherein n is preferably selected from the group of 1, 2, 3, 4 and 6.

More preferred are the inventive alkynyl ether compound of formula (I) wherein E is -CH₂-CH₂-C(O)- and n is 1 or 2 so that the inventive alkynyl ether compounds of formula (I) carry special functional groups selected from the group of mono- and divalent esters and mono- or divalent amides.

If n =1 or 2 the mono and divalent inventive alkynyl ether compounds of formula (I) are maintained.

If n=1 and the E-group is ethylene (-CH₂-CH₂-), Q is selected from the group of -CN, and substituted or unsubstituted 2-oxazolin 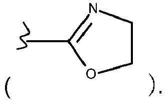 More preferred for n=1, E= -CH₂-CH₂- are the inventive compounds selected from the group of 2-cyanoethyl monoether of 2,5-hexynediol, unsubstituted 2-oxazolinethyl monoether and with C₁- C₄ alkyl or C₁-C₄ alkylhydroxid groups substituted 2-oxazolinethyl monoether of 1,4-butynediol and/or 2,5-hexynediol. Much more preferred for n=1 and E= - CH₂-CH₂- are the inventive compounds selected from the group of 3-(4-hydroxy-1-methyl-pent-2-ynoxy)propanenitrile, 4-[2-(4,5-dihydrooxazol-2-yl)ethoxy]but-2-yn-1-ol, 5-[2-(4,5-dihydrooxazol-2-yl)ethoxy]hex-3-yn-2-ol, 4-[2-[4-(hydroxymethyl)-4,5-dihydrooxazol-2-yl]ethoxy]but-2-yn-1-ol, 5-[2-[4-(hydroxymethyl)-4,5-dihydrooxazol-2-yl]ethoxy]hex-3-yn-2-ol, 4-[2-(4,4-dimethyl-5H-oxazol-2-yl)ethoxy]but-2-yn-1-ol and 5-[2-(4,4-dimethyl-5H-oxazol-2-yl)ethoxy]hex-3-yn-2-ol.

If n=2 and the E-group is ethylene (-CH₂-CH₂-), Q is selected from the group of a divalent sulfones, substituted or unsubstituted 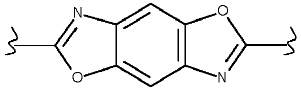 group and a divalent substituted or unsubstituted 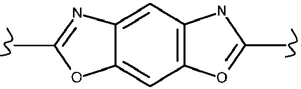 (benzobisoxazol-2,6-diyl) group.

Preferred is Q selected from the group of divalent sulfones selected from the group of 4-[2-[2-(4-hydroxybut-2-ynoxy)ethylsulfonyl]ethoxy]but-2-yn-1-ol and 5-[2-[2-(4-hydroxy-1-methylpent-2-ynoxy)ethylsulfonyl]ethoxy]hex-3-yn-2-ol, unsubstituted divalent 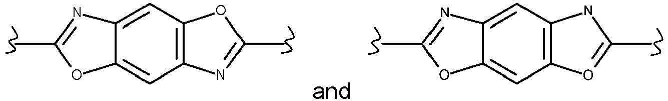 (benzobisoxazol-2,6-diyl) selected from the group of 4-[2-[2-[2-(4-hydroxybut-2-ynoxy)ethyl]oxazolo[4,5-f][1,3]benzoxazol-6-yl]ethoxy]but-2-yn-1-ol, 5-[2-[2-[2-(4-hydroxy-1-methyl-pent-2-ynoxy)ethyl]oxazolo[4,5-f][1,3]benzoxazol-6-yl]ethoxy]hex-3-yn-2-ol, 4-[2-[2-[2-(4-hydroxybut-2-ynoxy)ethyl]oxazolo[5,4-f][1,3]benzoxazol-6-yl]ethoxy]but-2-yn-1-ol and 5-[2-[2-[2-(4-hydroxy-1-methyl-pent-2-ynoxy)ethyl]oxazolo[5,4-f][1,3]benzoxazol-6-yl]ethoxy]hex-3-yn-2-ol, if n=2 and E is ethylene (-CH₂-CH₂-).

If n=1 or 2 and the E-group is 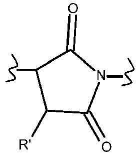 with R' which is selected from the group of H and a C₁-C₈ alkyl group, wherein the carbon atom of the terminal -CH- group next to the -CHR'- group of E is bonded to the -O- of formula (I) so that the maleimide group of E is a special embodiment of the inventive alkynyl ether compounds with an amide group as special functional group, Q is selected from the group of substituted or unsubstituted C₆-C₄₀ aryl groups, for n=1 and for n=2 Q is selected from the group of divalently bonded substituted or unsubstituted C₁₃-C₄₀ arylalkylenaryl groups and divalently bonded substituted or unsubstituted C₈-C₄₀ alkylenarylalkylen groups. Preferred is Q phenyl for n=1 and 1,3-Benzenedimethylen or bis (1,4-phenylen)methane and bis (2-Ethyl-5-methyl-1,4-phenylen)methane for n=2 and E =

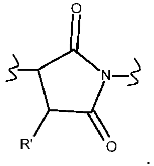

If n=1 or 2 and the E-group is -CH₂-CH₂-C(O)-, Q is selected from the group of -O-Y, -NR²-Y, as a special variation of NR²-Y the substituted or unsubstituted piperazin ring ( 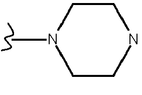 ), -O-Z-O-, -NR²-Z-NR²-, NR²-Z-O- and 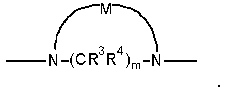 These inventive alkynyl ether compounds carry either an ester or an amide functional group.

If Q is the -O-Y or the -O-Z-O- group for n=1 or 2 and E = -CH₂-CH₂-C(O)- the inventive alkynyl ether compounds carry as special functional group an ester group.

If Q is the -NR²-Y group, a substituted or unsubstituted piperazin ring, -NR²-Z-NR²-, -NR²-Z-O- and 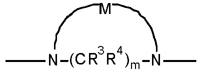 for n=1 or 2 and E = -CH₂-CH₂-C(O)-, the inventive alkynyl ether compounds carry as special functional group an amide group.

As well for the esters as for the amides Y is selected from the group of substituted or unsubstituted C₁-C₄₀ alkyl groups, substituted or unsubstituted saturated or unsaturated C₃-C₄₀ cycloalkyl groups, substituted or unsubstituted C₂-C₄₀ (poly)ether groups, substituted or unsubstituted saturated or unsaturated heterocyclic groups containing 2 to 20 carbon atoms and one or more heteroatoms selected from the group of N, O, and S as ring members, substituted or unsubstituted saturated or unsaturated alkylenheterocyclic groups containing 3 to 20 carbon atoms and one or more heteroatoms selected from the group of N, O, and S as ring members, substituted or unsubstituted C₆-C₄₀ aryl groups, substituted or unsubstituted C₂-C₄₀ alkenyl groups, substituted or unsubstituted C₂-C₄₀ alkynyl groups, substituted or unsubstituted saturated or unsaturated C₃-C₄₀ alkylencycloalkyl groups, substituted or unsubstituted C₇-C₄₀ alkylenaryl groups, substituted or unsubstituted α,β- unsaturated carbonyl groups containing C₃-C₄₀ atoms, substituted or unsubstituted C₃-C₁₉ keto groups and substituted or unsubstituted C₁-C₄ alkylen group which is part of a substituted or unsubstituted saturated heterocyclic group that is build up between R² of the -NR²- group and Y to form 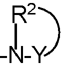 .

As well for the esters as for the amides Z is selected from the group of substituted or unsubstituted C₁-C₄₀ alkylen groups, divalently substituted or unsubstituted C₂-C₄₀ (poly)ether groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded substituted or unsubstituted alkylenheterocyclicalkylen groups containing 4 to 20 carbon atoms and one or more heteroatoms selected from the group of N, O, and S as ring members, divalently bonded substituted or unsubstituted C₆-C₄₀ aryl groups, divalently bonded substituted or unsubstituted C₇-C₄₀ cycloalkylalkylencycloalkyl groups, divalently bonded substituted or unsubstituted saturated or unsaturated C₅-C-₄₀ alkylencycloalkylalkylen groups, divalently bonded substituted or unsubstituted C₁₃-C₄₀ arylalkylenaryl groups, divalently bonded substituted or unsubstituted C₈-C₄₀ alkylenarylalkylen groups and divalently bonded substituted or unsubstituted C₁₇-C₃₀ alkylen-bisphenol A -alkylen groups.

In 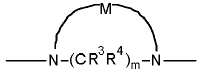 group the groups R³ and R⁴ are independently selected from the group of H and substituted or unsubstituted C₁-C₄₀ alkyl groups, while m is a natural number selected from the group of 1, 2 or 3 and M is selected from the group of substituted or unsubstituted C₁-C₃ alkylen groups, divalently bonded -NR⁵-CR³R⁴- groups, divalently bonded-NR⁵-NR⁶- groups, divalently bonded -NR⁵-CR³R⁴-CR³R⁴- groups, divalently bonded -O-CR³R⁴-CR³R⁴- groups, divalently bonded -S-CR³R⁴-CR³R⁴- groups, divalently bonded CR³R⁴-O-CR³R⁴ groups, divalently bonded -CR³R⁴-S-CR³R⁴- groups and divalently bonded-CR³R⁴-NR⁵-CR³R⁴- groups with R⁵ and R⁶ are independently selected from the group of H, substituted or unsubstituted C₁-C₄₀ alkyl groups, C₆-C₄₀ aryl groups, C₃-C₄₀ cycloalkyl groups and -C(O)CH=CH₂ group.

Preferred inventive alkynyl ether compounds with an ester functional group are selected from the group where n=1 or 2 and E = -CH₂-CH₂-C(O)- and wherein Y in the -O-Y group is selected from the group of substituted or unsubstituted C₁-C₄₀ alkyl groups, substituted or unsubstituted saturated or unsaturated C₃-C₄₀ cycloalkyl groups, substituted or unsubstituted saturated alkylenheterocyclic groups containing 3 to 20 carbon atoms and one or more heteroatoms selected from the group of N, O, and S as ring members, unsubstituted, saturated C₃-C₄₀ alkylencycloalkyl groups and

Z which is selected from the group of substituted or unsubstituted C₁-C₄₀ alkylen groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded saturated or unsaturated substituted or unsubstituted C₅-C₄₀ alkylencycloalkylalkylen groups and divalently bonded substituted or unsubstituted C₁₇-C₃₀ alkylen-bisphenol A- alkylen groups.

More preferred is Y in the -O-Y group for the inventive alkynyl ether compound with an ester functional group for n=1 selected from the group of substituted or unsubstituted C₁-C₁₀ alkyl groups, substituted or unsubstituted saturated or unsaturated C₄-C₁₁ cycloalkyl groups, substituted or unsubstituted saturated alkylenheterocyclic groups containing 3 to 10 carbon atoms and one or more O-atoms as heteroatom and as ring members, unsubstituted, saturated C₈-C₁₂ alkylencycloalkyl groups. Most preferred is Y in the -O-Y group for the inventive alkynyl ether compound with an ester functional group for n=1 selected from the group of tert.-butyl, n-propyl, 2,2-dimethylpropan-3-yl-1-ol, 2-hydroxylpropyl, isobornyl, bornyl, menthyl, terpinyl, dihydrodicyclopentadienyl, cyclopentanyl, cyclohexanyl, norbornyl, 3,3,5-Trimethylcyclohexanyl, 4-tert.-Butylcyclohexyl, 4-methylen-1,3-dioxolan, 4-methylen-2,2,dimethyl-1,3-dioxolan and 3-methylen-3-ethyl-1-oxetan.

More preferred is Z in the -O-Z-O- group for the inventive alkynyl ether compound with an ester functional group for n=2 selected from the group of substituted C₃-C₄₀ alkylen groups, divalently bonded substituted C₃-C₁₀ cycloalkyl groups, divalently bonded unsubstituted heterocyclic groups containing 3 to 10 carbon atoms and one or more -O-atoms as heteroatoms and as ring members, divalently bonded saturated unsubstituted C₅-C₁₄ alkylencycloalkylalkylen groups and divalently bonded unsubstituted C₁₇-C₂₅ alkylen-bisphenol A-alkylen groups. Most preferred is Z in the -O-Z-O- group for the inventive alkynyl ether compound with an ester functional group for n=2 selected from the group of 2,2-dimethyl-propan-1,3-diyl, 2,2,4,4-Tetramethylcyclobutan-1,3-diyl, Tricyclo[5.2.1.0^{2,6}]decan-4,8-dimethylen, hexahydro-furo[3,2-b]furan-3,6-diyl and Bisphenol A -bis(2-hydroxypropy-3-diyl)ether.

Preferred inventive alkynyl ether compounds with an amide functional group are selected from the group where n=1 or 2 and E = -CH₂-CH₂-C(O)- and wherein Y in the -NR²-Y group is selected from the group of substituted or unsubstituted C₁-C₄₀ alkyl groups and substituted or unsubstituted C₆-C₄₀ aryl groups with R² selected from the group of H and substituted or unsubstituted C₁-C₄₀ alkyl groups, and wherein a substituted or unsubstituted piperazin ring is a 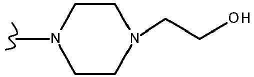 (4-(2-hydroxyethyl)piperazin-1-yl group and Z in the -NR²-Z-NR²- and -NR²-Z-O- group is selected from the group of substituted or unsubstituted C₁-C₄₀ alkylen groups, divalently bonded substituted or unsubstituted C₆-C₄₀ aryl groups and divalently bonded substituted or unsubstituted alkylenheterocyclicalkylen groups containing 4 to 20 carbon atoms and one or more heteroatoms selected from the group of N, O, and S as ring members, and wherein in the 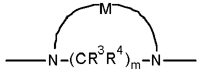 group R³ and R⁴ are independently selected from the group of H and substituted or unsubstituted C₁-C₁₀ alkyl groups, while m is a natural number selected from the group of 1 and 2 and M is selected from the group of substituted or unsubstituted C₁-C₃ alkylen groups and divalently bonded -CR³R⁴-NR⁵-CR³R⁴- groups with R⁵ and R⁶ are independently selected from the group of H, substituted or unsubstituted C₁-C₄₀ alkyl groups, C₆-C₄₀ aryl groups, C₃-C₄₀ cycloalkyl groups and -C(O)CH=CH₂ group. More preferred is Y in the -NR²-Y group for the inventive alkynyl ether compound with an amide functional group for n=1 selected from the group of OH-substituted C₂-C₅ alkyl group, keto- and alkyl-substituted C₃-C₁₀ alkyl group, OH-substituted C₆-C₄₀ aryl groups. Most preferred is Y in the -NR²-Y group for the inventive alkynyl ether compound with an amide functional group for n=1 selected from the group of methylhydroxid, eth-2yl-hydroxid, 2-Methylpropan-2yl-3- ol, 2-Hydroxypropyl, 2-methoxy-1,1-dimethylethyl and p-Hydroxyphenyl, with R² selected from the group of H and 2-Hydroxypropyl.

More preferred is Z in the -NR²-Z-NR²- and -NR²-Z-O- group for the inventive alkynyl ether compound with an amide functional group for n=2 selected from the group of unsubstituted C₁-C₄₀ alkylen groups, divalently bonded substituted or unsubstituted C₆-C₄₀ aryl groups and divalently bonded unsubstituted alkylenheterocyclicalkylen groups containing 4 to 20 carbon atoms and one or more heteroatoms selected from the group of N, O, and S as ring members. Most preferred is Z in the -NR²-Z-NR²- and -NR²-Z-O- group for the inventive alkynyl ether compound with an amide functional group for n=2 selected from the group of methylen, ethylene, 1,3-phenyl, 2,5-Dihydroxy-1,4-phenyl, 2,5-dimethylentetrahydrofuran and 2,5-dimethylenfuran with R²= H.

More preferred is M = -CH₂-CH₂- and R³ and R⁴= H with m= 2 in the 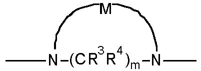 group so that it is a 1,4-diazinanyl (piperazinyl) group.

Preferred inventive alkynyl ether compounds of formula (I) with multivalent ester or amide groups as special functional groups carry an -CH₂-CH₂-C(O)- group as E, n is selected from the group of 3 and ≥ 4 and Q is selected from the group of a substituted or unsubstituted 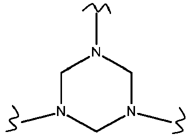 (1,3,5-triazinan) group, a 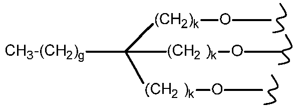 group, a 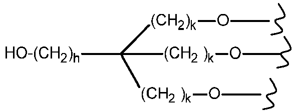 group, a 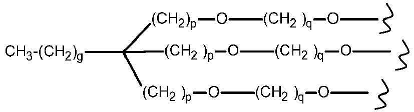 group, 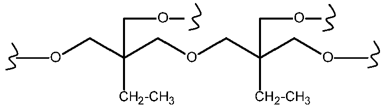 group and 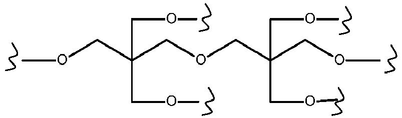 group, wherein
- g: is a natural number selected from 1 to 33,
- k: is a natural number selected from 1 to 12,
- h: is a natural number selected from 1 to 15,
- p: is a natural number selected from 1 to 3 and
- q: is a natural number selected from 1 to 5, wherein the sum of C-atoms in the groups with g, k, h, p and q is not higher than 40.

For n=3, Q is preferably selected from the group of with three -C(O)-CH₂-CH₂-O-CHR¹-C≡C-CHR¹-OH groups substituted 1,3,5-triazinan, the group where g= 1-4 and k = 1-3, the group where h =1-15 and k =1-3 and the group where g = 1-4, p = 1-3 and q =1-3. More preferably for n=3, Q is selected from the group of with three -C(O)-CH₂-CH₂-O-CHR¹-C≡C-CHR¹-OH groups substituted 1,3,5-triazinan, the group where g=1 and k=1, the group where h= 1, k=1, the group where g=1, p=1 and q=3.

For n≥ 4, preferably n = 4 or 6, Q is preferably selected from the group of

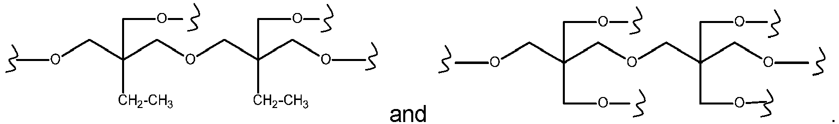

In order to produce the inventive alkynyl ether compounds of formula (I) an α,β- unsaturated functional compound selected from the group of compounds of formula (II) 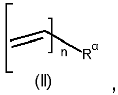 acrylonitrile (NC-CH=CH₂), maleinimide-N-Q-group 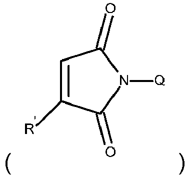 and maleinimide-N-Q-N-maleininimide group 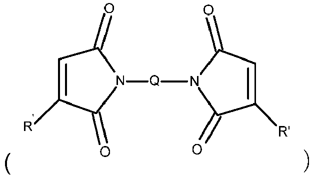 with Q as defined above for the case where n is 1 or 2 and E is 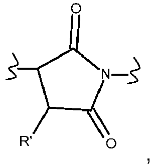 reacts with 1,4,-butynediol and/or 2,5-hexynediol, wherein for n = 1 R^{α} is selected from the group of -CN, 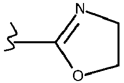 (2-oxazolin), -C(O)-O-Y and -C(O)-NR²-Y, for n = 2 R^{α} is selected from the group of -C(O)-A-C(O)-, -S(O)₂- and 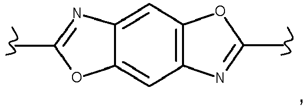 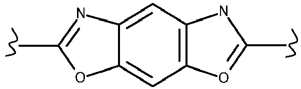 (benzobis oxazol-2,6-diyl), for n = 3 R^{α} is selected from the group of 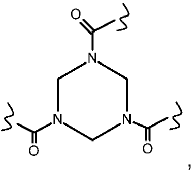 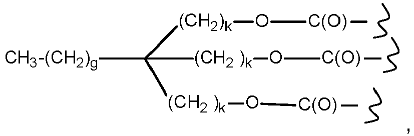 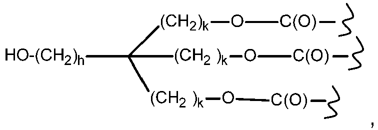 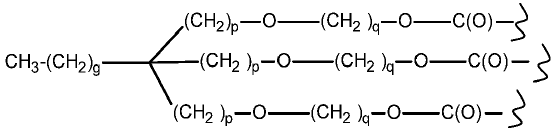 wherein g = 1-33, k = 1-12, p = 1-3 and q = 1-5, for n ≥ 4 R^{α} is selected from the group of 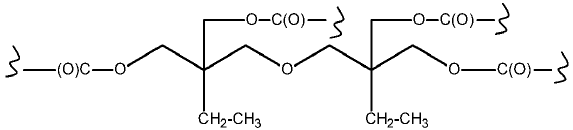 and 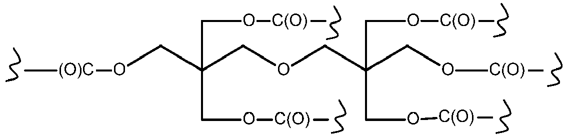 and where R', Y, R² and A are as defined above.

A further embodiment of the invention is an alternative process for the preparation of the inventive alkynyl ether compound of formula (I) where E= -CH₂-CH₂-C(O)- and Q is selected from the group of NR²-Y and -X¹-Z-X²-, wherein the compounds where X¹ and X² are both O are disclaimed 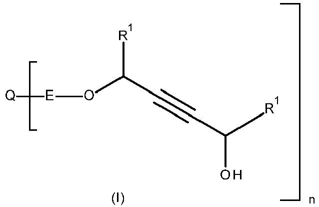 comprising the following steps:
a) reacting an α,β- unsaturated carbonyl compound of formula (Ila) 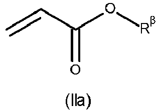
   with R^{β} selected from the group of C₃-C₁₀ alkyl groups,
   with 1,4,-butynediol and/or 2,5-hexynediol,
   wherein the resulting product of step a), which is the ester of formula (III) or a mixture of two E/Z-isomers of the ester of formula (III), 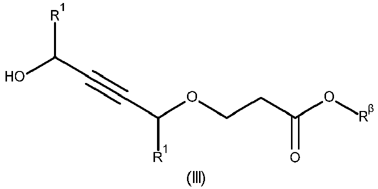
b) reacts in a further step b) with a compound selected from the group of amine, diamine, triamine, substituted or unsubstituted ethanolamine, alcohol, dialcohol and multi alcohol, wherein the multi alcohol comprises 3 or more OH groups,
wherein R¹, Y, R², Z and n are as defined above.

In the alternative inventive process the resulting product of step a) reacts with at least one N-atom of a compound selected from the group of HNR²-Y, H₂N-Z-NH₂, N-substituted piperazine, substituted or unsubstituted 1,3,5-triazinane, H₂N-Z-OH,
wherein Y, R², Z and n are as defined above.

All inventive alkynyl ether compounds are directly received after the reaction of the α,β- unsaturated functional compound of formula (II) with 1,4-butynediol and/or 2,5-hexynediol, hereinafter referred to as diol. Preferred the monoesters of the inventive alkynyl ether compound of formula (I) with Q selected from the group of -C₃-C₁₀ alkyl groups and E = -CH₂-CH₂-(CO)- are produced via the inventive process. More preferably the n-propyl-, iso-propyl-, n-butyl-, sec-butyl-, tert.-butyloxycarbonyl)ethylether of 2-butyne-1,4-diol and/or 2-hexyn-2,5-diol will be produced via the inventive process. Most preferably the propyl 3-(4-hydroxybut-2-ynoxy)propanoate, propyl 3-(4-hydroxy-1-methyl-pent-2-ynoxy)propanoate, tert-butyl 3-(4-hydroxybut-2-ynoxy)propanoate and tert-butyl 3-(4-hydroxy-1-methyl-pent-2-ynoxy)propanoate are produced via the inventive process.

To increase selectivity of the inventive alkynyl ether compounds where n is 1, it is preferred that to the 1,4-butynediol and/or 2,5-hexynediol, hereinafter referred to as diol, the α,β- unsaturated functional compound of formula (II) is added.

It is preferably that during the reaction of the α,β- unsaturated functional compound of formula (II) or the α,β- unsaturated carbonyl compound of formula (IIa) with 1,4-butynediol and/or 2,5-hexynediol of the inventive or alternative process the molar ratio between the diol and the α,β- unsaturated functional compound of formula (II) or the α,β- unsaturated carbonyl compound of formula (Ila) is in the range from 1:1 to 10:1, more preferably in the range from 1:1 to 3:1, much more preferably in the range from 1:1 to 2:1, most preferably in the range from 1:1 to 1.5:1.

The temperature at normal pressure during the reaction of the diol with the α,β- unsaturated functional compound of formula (II) or the or the α,β- unsaturated carbonyl compound of formula (Ila) will be in the range from 25°C to 120°C, preferably in the range from 30 to 110°C, more preferably in the range from 30 to 100°C, much more preferably in the range from 30 to 80°C.

Preferably the reaction of the diol with the α,β- unsaturated functional compound of formula (II) or the α,β- unsaturated carbonyl compound of formula (Ila) uses a catalyst. This catalyst is selected of the group of para toluene sulfonic acid, trifluor acetic acid, methane sulfonic acid, (CF₃SO₂)₂NH, trialkyl phosphines, azaphosphatrenes, KF/Al₂O₃, BF₃ OEt₂, La(NO₃)₃, Ce(NO₃)₃, Zn(OTf)₂, Sn(OTf)₂, Sn(OTf)₄, In(OTf)₃, Al(OTf)₃, biphenyldiamine- or N-heterocyclic carbenes, alkali or alkaline earth metal hydroxide carbonate, tertiary amines, amidine, imidazole, bicyclic guanidine, substituted imidazole compounds, substituted pyrrolidine, substituted or unsubstituted piperidine, pyridine and substitutes pyridines, phase transfer catalysts, quaternary phosphonium salts and ammoniumhydroxide, sodium, potassium and caesium hydroxide and carbonate, N.N-dimethylethanolamine (DMEA), diaminobicyclooctane (DABCO), triethylene diamine (TEDA), bis(2-dimethylaminoethyl)ether (BDMAEE), N-ethylmorpholine, N'N'-dimethylpiperazine, N.N.N',N',N'-pentamethyl-diethylene-tri amine (PMDETA), N,N'-dimethylcyclohexylamine (DMCHA), N,N-dimethylbenzylamine (DMBA)₃, , N,N-dimethylethylamine, N.N.N',N',N'-pentamethyl-dipropylene triamine (PMDPTA), triethylamine, tripropylamine, tributylamine, tripentylamine, trihexylamine, trioctylamine, 1-(2-hydroxypropyl)imidazole, tetramethylguanidine (TMG), diz abicyclononene (DBN) and diazabicyclo undecene (DBU), 1.5.7.-triazabi cyclo4.4.0dec-5-ene (TBD), 1.5.7.-triazabicyclo4.4.0 dec-5-ene, 7-methyl (MTBD), 1,4,5,6- tetrahydropyrimidine, dimethylamino pyridine (DMAP), tetrakis hydroxyethyl ammonium hydroxide, tetraethyl ammonium hydroxide, tetramethyl ammonium hydroxide, tetrapropyl ammonium hydroxide, tetraisopropyl ammonium hydroxide, tetrabutyl ammonium hydroxide, tetraisobutyl ammonium hydroxide, tetra-tert.-butyl ammonium hydroxide, tetrapentyl ammonium hydroxide, tetrahexyl ammonium hydroxide, tetraheptyl ammonium hydroxide, tetraoctyl ammonium hydroxide, benzyltrimethyl ammonium hydroxide, diethyldimethyl ammonium hydroxide, methyltripropyl ammoniumydroxide, N,N,N,N',N',N'-hexabutylhexamethylen diammonium hydroxide, tetrakis(2-hydroxyethyl) ammonium hydoxide, tributylmethy lammonium hydroxide, tributylmethyl ammonium hydroxide, triethylmethyl ammonium hydroxide, trimethylphenyl ammonium hydroxide, (2-hydroxyethyl)trimethyl ammonium hydroxide, (2-hydroxyethyl)triethyl ammonium hydroxide, (2-hydroxyethyl)tripropyl ammonium hydroxide, (2-hydroxyethyl)tributyl ammonium hydroxide, hexamethyl ammonium hydroxide, dimethyldiethanol ammonium hydroxide or mixtures thereof, each as waterfree solids, in different grades of hydrations as solid, dissolved in aqueous or non-aqueous solvents or mixtures thereof, adsorbed or covalently attached to support materials or as dispersions,

The inventive process as well as the alternative process to produce the inventive alkynyl ether compounds of formula (I) can be operated in the presence of no solvent or in the presence of a solvent. This depends on the solubility properties of the 1,4-butynediol and/or 2,5-hexynediol and the used α,β-unsaturated functional compound of formula (II) or α,β-unsaturated carbonyl compound of formula (Ila). Is at least one or the reaction partners the alkynol and/or Michael system) in liquid form at reaction temperature, no solvent might be required. Is the reaction temperature above the melting point of the used α,β-unsaturated functional compound of formula (II) or α,β-unsaturated carbonyl compound of formula (IIa) and the α,β-unsaturated functional compound of formula (II) or α,β-unsaturated carbonyl compound of formula (IIa) is dissolvable in the 1,4-butynediol and/or 2,5-hexynediol, then the process does not need any solvent. No solvents are more preferable needed, if the molar ratio between 1,4-butyndiol and/or 2,5-hexyndiol to α,β-unsaturated functional compound of formula (II) or α,β-unsaturated carbonyl compound of formula (IIa) is higher than 1:1, preferable higher than 3:1 and the reaction temperature is above the melting point of 1,4-butyndiol and/or 2,5-hexyndiol and if the reaction product formed is not soluble in water. In this version of the inventive process the excess of 1,4-butynediol and/or 2,5-hexyndiol can be washed out with water after the reaction of α,β-unsaturated functional compound of formula (II) or α,β-unsaturated carbonyl compound of formula (Ila) with the 1,4-butynediol and/or 2,5-hexynediol. As a result the desired alkynyl ether compound of formula (I) will be received without using any solvent.

A further alternative solvent for the inventive or alternative inventive process for the production of the inventive alkynyl ether compounds of formula (I) is the use of a polar organic solvent or mixtures of solvents. A polar organic solvent means a solvent that is selected from the group of (cyclic) ethers such as THF or dioxane, acetonitrile, lactones such as valerolactone, ketaly such as Dihydrolevoglucosenon, DMF, NMP and DMSO. Preferably are the polar organic solvents THF, dioxane and acetonitrile.

A further alternative solvent for the inventive or alternative inventive process for the production of the inventive alkynyl ether compounds of formula (I) is the use of an aqueous solvent. An aqueous solvent means a polar organic solvent together with water or water alone. If an aqueous solvent is used it is preferable if the molar ration between 1,4-butynediol and/or 2,5-hexynediol and α,β-unsaturated functional compound or α,β-unsaturated carbonyl compound is in the range of 1:1 to 3:1, more preferable in the ratio of 1:1 to 1.5:1. More preferably is water alone as solely aqueous solvent for the reaction of between 1,4-butynediol and/or 2,5-hexynediol and α,β-unsaturated functional compound or α,β-unsaturated carbonyl compound in the above mentioned range. The resulting aqueous mixture of the reaction of 1,4-butynediol and/or 2,5-hexynediol and α,β-unsaturated functional compound or α,β-unsaturated carbonyl compound in water as solely solvent is preferable to use as corrosion protection agent. Another further alternative inventive process for the production of the inventive alkynyl ether compounds of formula (I) is to dissolve the α,β-unsaturated functional compound of formula (II) or α,β-unsaturated carbonyl compound of formula (IIa) in a polar organic solvent and to add this mixture to the 1,4-butynediol and/or 2,5-hexynediol which is dissolved in water.

With all these alternative solvents for the inventive and alternative inventive processes the inventive alkynyl ether compound of formula (I) is received either as dissolved in a solvent or dissolved in an excess of 1,4-butynediol and/or 2,5-hexynediol. For the use as corrosion protection agent it will not be necessary to isolate the inventive alkynyl ether compound of formula (I) of the solvent. It is preferable to remove the solvent as well as the excess of diol and after additional purification the inventive alkynyl ether compound of formula (I) is in order to use as corrosion protection agent as well as starting product for the preparation of cyclic carbonates with exocyclic vinylidene group of formula (IV).

### Examples

### Example 1

### Synthesis of the tert-Butyl-BYD adduct (tBuA-BYD) (tert.-Butyl-3-(4-hydroxybut-2-ynoxy)propanoate) in solution

2-Butyne-1,4-diol (537 g, 6.24 mol, 10.0 eq.) was placed in a 2 L three-neck flask and dissolved in 600 mL 1,4-dioxane. The mixture was heated to 60 °C. When 2-butyne-1,4-diol was completely dissolved, DBU (19.0 g, 125 mmol, 18.6 mL, 0.20 eq.) was added. *tert*-Butyl acrylate (80.0 g, 624 mmol, 91.0 mL, 1.00 eq.) was mixed with 100 mL 1,4-dioxane and was added *via* dropping funnel over a period of 1 h. The reaction mixture was then stirred at 60 °C for 24 h. The solvent was removed under reduced pressure. The residue was dissolved in a mixture of water and ethyl acetate (1:1 v/v, 600 mL). The aqueous phase was separated and extracted twice with ethyl acetate (2 * 200 mL). The combined organic phases were then washed with distilled water (4 * 500 mL). The organic phase was dried over sodium sulfate and the solvent was removed under reduced pressure. The product tBuA-BYD (tert.-Butyl-3-(4-hydroxybut-2-ynoxy)propanoate) was obtained as yellow-brown liquid. It was redissolved in 200 mL acetonitrile, treated with activated carbon, filtered over a silica plug and isolated as a yellow liquid (108 g, 625 mmol, 81%).

### Example 2

### Synthesis of the tert-Butyl-BYD adduct (BuA-BYD) as a melt

2-Butyne-1,4-diol (3.36 g, 39.0 mmol, 10.0 eq.) was placed in a 50 mL flask and heated to 70 °C. When 2-butyne-1,4-diol was liquefied, DBU (119 mg, 780 µmol, 116 µL 0.20 eq.) was added. *tert*-Butyl acrylate (500 mg, 3.90 mmol, 568 µL, 1.00 eq.) was added dropwise *via* syringe. The reaction mixture was then stirred at 70 °C for 24 h. The hot melt was dissolved in a mixture of water and ethyl acetate (1:1 v/v, 20 mL). The aqueous phase was separated and extracted twice with ethyl acetate (2 * 20 mL). The combined organic phases were then washed with distilled water (4 * 50 mL). The organic phase was dried over sodium sulfate and the solvent was removed under reduced pressure. The product tBuA-BYD (tert.-Butyl-3-(4-hydroxybut-2-nyoxy)propanoate) was obtained as yellow-brown liquid. It was redissolved in 200 mL acetonitrile, treated with activated carbon, filtered over a silica plug and isolated as a yellow liquid (460 mg, 2.15 mmol, 55%).

**¹H-NMR** (300 MHz, CDCl₃):
*δ* [ppm] = 4.23 (d, *J* = 5.8 Hz, 2 H, C*H*₂), 4.12 (s, 2 H, C*H*₂), 3.68 (t, *J* = 6.4 Hz, 2 H, O*H*),
3.09 (t, *J* = 5.8 Hz, 1 H, C*H*₂), 2.45 (t, *J* = 6.4 Hz, 2 H, C*H*₂), 1.38 (s, 9 H, C*H*₃).

**¹³C-NMR** (75 MHz, CDCl₃):
*δ* [ppm] = 171.2 (*C*_{Carbonyl}), 85.2 (*C*_{alkyne}), 81.3 (*C*_{q}), 81.1 (*C*_{alkyne}), 65.1 (*C*_{alkyl}), 58.6 (*C*_{alkyl}), 50.9 (*C*_{alkyl}), 36.2 (*C*_{alkyl}), 28.2 (*C*_{Me}).

**HR-MS** (APCI, pos):
Calculated for C₁₁H₂₂O₄N: m/z = 232.1543 [M+NH₄⁺]
Found: m/z = 232.1545 correct isotope distribution

### Example 3

### Synthesis of the acrylonitrile-HYD adduct (3-(4-Hydroxy-1-methyl-pent-2-ynoxy)propanenitrile) in solution

3-Hexyne-2,5-diol (4.30 g, 37.7 mmol, 10.0 eq.) was placed in a 50 mL flask and mixed with 5 mL 1,4-dioxane. The mixture was heated to 60 °C. DBU (115 mg, 753 pmol, 110 µL, 0.20 eq.) was added. Acrylonitrile (200 mg, 250 µL, 1.00 eq.) was added dropwise *via* syringe. The reaction mixture was then stirred at 60 °C for 24 h. The solvent was removed under reduced pressure. The residue was dissolved in a mixture of water and ethyl acetate (1:1 v/v, 20 mL). The aqueous phase was separated and extracted twice with ethyl acetate (2 * 20 mL). The combined organic phases were then washed with distilled water (4 * 25 mL). The organic phase was dried over sodium sulfate and the solvent was removed under reduced pressure. The crude product (3-(4-Hydroxy-1-methyl-pent-2-ynoxy)propanenitrile) was obtained as yellow-brown liquid. It was dissolved in 20 mL acetonitrile, treated with activated carbon, filtered over a silica plug and isolated as a yellow liquid (200 mg, 1.20 mmol, 31%).

**¹H-NMR** (300 MHz, CDCl₃):
*δ* [ppm] = 4.57-4.51 (m, 1 H), 4.27-4.20 (m, 1H), 3.92-3.85 (m, 1 H), 6.64-3.57 (m, 1H), 2.61 (t, *J* = 6.3 Hz, 2H), 1.42 (dd, *J* = 6.5, 5.2 Hz, 6 H).

**¹³C-NMR** (75 MHz, CDCl₃):
*δ* [ppm] = 118.3 (*C*ₙₜᵣᵢₗₑ), 88.1 (*C*_{alkyne}), 82.9 (*C*_{alkyne}), 66.2 (*C*_{alkyl}), 63.3 (*C*_{alkyl}), 58.4 (*C*_{alkyl}), 24.6 (*C*_{methyl}), 22.2 (*C*_{alkyl}), 19.2 (*C*_{alkyl}).

**HR-MS** (ESI, pos):
Calculated for C₉H₁₃O₂NNa: m/z = 190.0838 [M+Na]
Found: m/z = 243.1104 C₁₂H₁₆O₂N₂Na

### Example 4

### Synthesis of the 1,3,5-triacryloylhexahydro-1,3,5-triazine-BYD adduct (1-[3,5-Bis[3-(4-hydroxybut-2-ynoxy)propanoyl]-1,3,5-triazinan-1-yl]-3-prop-2-ynoxy-propan-1-one) in solution

2-Butyne-1,4-diol (104 g, 1.20 mol, 10.0 eq.) was placed in a 500 mL flask and dissolved in 150 mL 1,4-dioxane. The mixture was heated to 60 °C. When 2-butyne-1,4-diol was completely dissolved, DBU (3.66 g, 24.1 mmol, 3.60 mL, 0.20 eq.) was added. 1,3,5-Triacryloylhexahydro-1,3,5-triazin (30.0 g, 120 mmol, 1.00 eq.) was mixed with 100 mL 1,4-dioxane and was added portion wise over a period over 20 minutes. The reaction mixture was then stirred at 60 °C for 24 h. The solvent was removed under reduced pressure. The residue was mixed with ethyl acetate (200 mL). The product separates itself from the ethyl acetate phase. The product phase was then washed with ethyl acetate (4 * 200 mL). The crude product was dried under reduced pressure. The product (1-[3,5-Bis[3-(4-hydroxybut-2-ynoxy)propanoyl]-1,3,5-triazinan-1-yl]-3-prop-2-ynoxy-propan-1-one) was obtained as reddish-brown liquid. It was dissolved in 200 mL acetonitrile, treated with activated carbon, filtered over a silica plug and isolated as a brown, viscous liquid (45.1 g, 88.9 mmol, 74%).

**¹H-NMR** (300 MHz, MeOD):
*δ* [ppm] = 5.46 (bs, 6 H, C*H*₂), 4.31-4.28 (m, 12 H, C*H*₂), 3.88 (bs, 6 H, C*H*₂), 2.93 (bs, 6 H C*H*₂).

**¹³C-NMR** (75 MHz, MeOD):
*δ* [ppm] = 172.2 (*C*_{carbonyl}), 86.2 (*C*_{alkyne}), 84.3 (*C*H₂), 81.6 (*C*_{alkyne}), 66.6 (*C*H₂), 59.2 (*C*H₂), 50.7 (*C*H₂), 34.1 (*C*H₂).

**HR-MS** (ESI, pos):
Calculated for C₂₄H₃O₉N₃Na: m/z = 530.2109 [M+Na⁺]
Found: m/z = 530.2112 correct isotope distribution

### Example 5

### Synthesis of (2,2-dimethyl-1,3-dioxolan-4-yl)methyl 3-((4-hydroxybut-2-yn-1-yl)oxy)propanoate

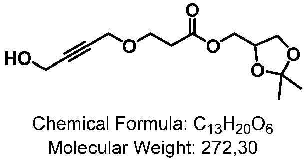

In a 1 L three-necked round-bottomed flask, charged with a large stir bar, dissolved 2-butyne-1,4-diol (BYD, 231 g, 2683.2 mmol, 9.3 equiv.) in 1,4-dioxane (300 mL) while stirring at 60 °C. When BYD was completely dissolved, BL120 (10 mL, 56.3 mmol, 20 mol%) was added dropwise over a period of ~5 min. Slowly added (2,2-dimethyl-1,3-dioxolan-4-yl)methyl acrylate (50 mL, 287.3 mmol, 1.0 equiv.), dissolved in 1,4-dioxane (70 mL) over a period of ~2 h. Let stir to 60 °C for 7 d. After cooling to room temperature, concentrated reaction solution in vacuum. The residue was uptaken in EtOAc and H₂O/brine. Extracted with EtOAc 3 times. Combined org. layers were washed with H₂O and brine, before drying over Na₂SO₄ and concentrating in vacuum, then purified via flash chromatography on SiO₂ using isohexane/EtOAc (4:1, 2:1, 1:3) as eluent. The titled product was obtained in high purity as a light-yellow, highly-viscous oil.

**¹H NMR** (300 MHz, CDCl₃): δ 4.34 - 4.26 (m, 1H), 4.25 (t, *J* = 1.8 Hz, 2H), 4.17 - 4.15 (m, 2H), 4.22 - 4.08 (m, 2H), 4.09 - 4.02 (m, 1H), 3.80 - 3.68 (m, 3H), 2.66 (s, 1H), 2.62 (t, *J* = 6.2 Hz, 2H), 1.40 (s, 3H), 1.35 - 1.32 (m, 3H).

**¹³C{¹H} NMR** (75 MHz, CDCl₃): δ 171.4, 109.9, 85.2, 81.2, 73.6, 66.3, 65.2, 64.8, 58.6, 50.9, 34.9, 26.7, 25.4.

**R_{f}** (isohexane/EtOAc; 1:1) = 0.33 [KMnO₄]

**HRMS (ESI):** [m/z] calculated for C₁₅H₁₅N₃O₆H⁺ ([M+H]⁺ ): 334.1034; Found: 334.1037.

**HRMS (ESI):** [m/z] calculated for C₁₅H₁₅N₃O₆Na⁺ ([M+Na]⁺ ): 356.0853; Found: 356.0857

### Example 6

Synthesis of 1,1'-(piperazine-1,4-diyl)bis(3-((4-hydroxybut-2-yn-1-yl)oxy)propan-1-one)

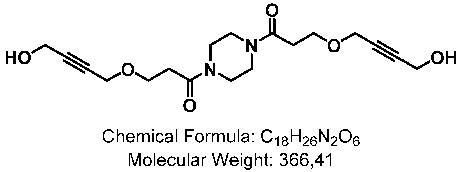

A 20 mL vial was charged with 2-butyne-1,4-diol (BYD, 2.12 g, 24.6 mmol, 10.2 equiv.), 1,4-dioxane (5 mL) and a stir bar, and then sealed with a cap through which a needle was put. Stirred at 50 °C for 5 min, before helping to dissolve BYD by adding DBU (80 µL, 0.54 mmol, 22 mol%) all at once. To this clear solution, added piperazine diacrylamide (0.47 g, 2.4 mmol, 1.0 equiv.). Continued stirring to 50 °C for 4 days. Combined two identically set-up reactions and purified via flash chromatography: Suspended raw product in DCM and added on top of silica-layer. Washed down first fraction (BYD) with pure MeCN. Product was eluated using pure EtOH and/or MeOH/DCM (1:1). Product containing fractions were again purified via flash chromatography using pure EtOH as eluent. The titled product was obtained as a white solid in 45% combined yield (81 mg, 0.223 mmol).

**¹H NMR** (300 MHz, MeCN-d₃): δ 4.17 (d, *J* = 1.7 Hz, 4H), 4.15 (d, *J* = 1.7 Hz, 4H), 3.73 (td, *J* = 6.5, 3.3 Hz, 4H), 3.51 (m, 8H), 2.60 (t, *J* = 6.5 Hz, 4H).

**¹³C{¹H} NMR** (75 MHz, MeCN-d₃): δ 170.4, 86.2, 81.5, 66.8, 58.9, 50.6, 33.9.

**¹H NMR** (300 MHz, DMSO-d₆): δ 5.23 (s, 2H), 4.15 (t, *J* = 1.8 Hz, 4H), 4.11 (t, *J* = 1.8 Hz, 4H), 3.67 (t, *J* = 6.6 Hz, 4H), 3.48 (s, 4H), 3.43 (d, *J* = 5.0 Hz, 4H), 2.61 (t, *J* = 6.6 Hz, 4H). **¹³C{¹H} NMR** (75 MHz, DMSO-d₆): δ 168.9, 86.2, 80.3, 65.6, 57.7, 49.0, 32.6.

**R_{f}** (EtOH) = 0.33 [KMnO₄]

**HRMS (ESI):** [m/z] calculated for C₁₈H₂₆N₂O₆H⁺ ([M+H]⁺ ): 367.1864; Found: 367.1862.

**HRMS (ESI):** [m/z] calculated for C₁₈H₂₆N₂O₆Na⁺ ([M+Na]⁺ ): 389.1683; Found: 389.1681.

### Example 7

Synthesis of *N*,*N*'-methylenebis(3-((4-hydroxybut-2-yn-1-yl)oxy)propanamide)

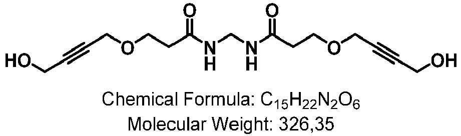

In a 1 L three-necked round-bottomed flask, charged with a large stir bar, dissolved 2-butyne-1,4-diol (BYD, 301.2 g, 3498.7 mmol, 11.4 equiv.) in 1,4-dioxane (350 mL) while stirring at 60 °C. When BYD was completely dissolved, DBU (10.5 mL, 70.3 mmol, 23 mol%) was added dropwise over a period of ~5 min. The reaction solution turned dark-red. Slowly added solid *N*,*N*'-methylenediacrylamide (47.5 g, 308.1 mmol, 1.0 equiv.) over a period of ∼20 min. Let stir to 60 °C for 48 h. A beige-precipitate was formed. After cooling to room temperature, let suspension stand to room temperature for 2.5 days. Filtered suspension and washed solid with ∼2 L H₂O, ∼500 mL EtOAc and ∼500 mL acetone. Let dry beige-white solid to open-air for several days, before drying in vacuum. The product was afforded as a rockhard, beige solid in 40% yield (40.0 g, 122.4 mmol).

**¹H NMR** (300 MHz, DMSO-d₆): δ 8.47 (t, *J* = 6.0 Hz, 2H), 5.20 (s, 2H), 4.37 (t, *J* = 6.0 Hz, 2H), 4.17 - 4.04 (m, 8H), 3.61 (t, *J* = 6.4 Hz, 4H), 2.33 (t, *J* = 6.4 Hz, 4H).

**¹³C{¹H} NMR** (75 MHz, DMSO-d₆): δ 170.4, 86.2, 80.3, 65.5, 57.6, 49.0, 43.2, 35.5.

**HRMS (ESI):** [m/z] calculated for C₁₅H₂₂N₂O₆H⁺ ([M+H]⁺): 327.1551; Found: 327.1555.

### Procedure for corrosion inhibition tests

Steel stripe parameters: zinc phosphate coated steel plates (GARDOBOND 2828T 68000G) 190 mm x 105 mm x 1 mm. Cut into stripes each 105 mm x 25 mm x 1 mm
1. Steel stripes were each washed with distilled water (50 mL) and second with isopropanol (25 mL).
2. The washed stripes were dried at 50 °C for 15 minutes, cooled down for 2 minutes to room temperature and weight afterwards.
3. A 250 mL beaker was filled with 100 mL of half concentrated hydrochloric acid.
   892 µmol of a propargylic alcohol (c = 8.92 mmol/L) were added and dissolved.
4. Steel stripe is placed in the beaker. The surface contact area was kept constant within the individual tests. The specimen were kept immersed for 1.5 h or 3 h at room temperature.
5. The steel stripe is removed from the beaker and washed with distilled water (50 mL) and isopropanol (25 mL).
6. The washed stripes were dried at 50 °C for 15 minutes, cooled down for 2 minutes to room temperature and weight afterwards.

Table below shows the mass data for the individual experiments before and after the corrosion test as well as the deviation in [%]

| **Additive [892 µmol]** | **m [g]before** | **m [g] after** | **Deviation [%]** |
|---|---|---|---|
| *N*,*N'*-methylenebis(3-((4-hydroxybut-2-yn-1-yl)oxy)propanamide) | 13,5910 | 13,5748 | 0,12 |
| 3-(4-hydroxybut-2-ynoxy)propanenitrile | 13,5960 | 13,5797 | 0,12 |
| tert-butyl 3-(4-hydroxybut-2-ynoxy)propanoate | 13,5750 | 13,5528 | 0,16 |
| none | 13,6133 | 13,4793 | 0,98 |
| Propargyl alcohol | 13,6330 | 13,6206 | 0,09 |

## Claims

1. Alkynyl ether compounds of formula (I) 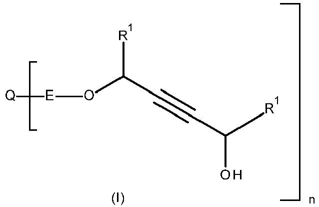 with
n is a natural number selected from the 1 to 10,
R¹ is selected from the group of H and methyl,
E is selected from the group of -CH₂-CH₂-, -CH₂-CH₂-C(O)-, and 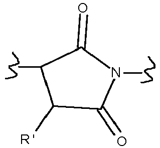
with R' is selected from the group of H and a C₁-C₈ alkyl group, wherein the carbon atom of the terminal -CH₂- or the -CH- group next to the-CHR'- group of E is bonded to the -O- of formula (I)
Q is dependent on n and E
for n=1 and E= -CH₂-CH₂-
Q is selected from the group of -CN and substituted or unsubstituted 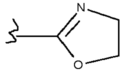 (2-oxazolin) group,
for n=1 and E= -CH₂-CH₂-C(O)-
Q is selected from the group of -O-Y, -NR²-Y and substituted or unsubstituted 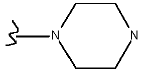 (piperazine), for n=1 and E= 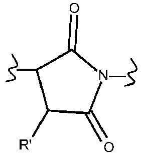
Q is selected from the group of substituted or unsubstituted C₆-C₄₀ aryl groups, for n=2 and E= -CH₂-CH₂-
Q is selected from the group of a divalent bonded sulfone (-S(O)₂-) group, a divalent substituted or unsubstituted 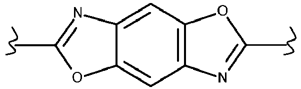 group and a divalent substituted or unsubstituted 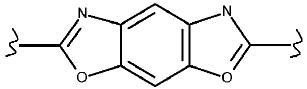 (benzobisoxazol-2,6-diyl) group,
for n=2 and E= -CH₂-CH₂-C(O)-
Q is a divalent -A- group,
for n=2 and E= 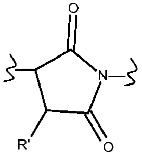
Q is selected from the group of divalently bonded substituted or unsubstituted C₄-C₁₀ alkylen groups, divalently bonded substituted or unsubstituted phenyl, divalently bonded substituted or unsubstituted C₁₃-C₄₀ arylalkylenaryl groups and divalently bonded substituted or unsubstituted C₈-C₄₀ alkylenarylalkylen groups,
for n=3 and E= -CH₂-CH₂-C(O)-
Q is selected from the group of a substituted or unsubstituted 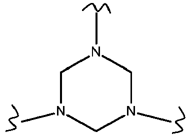 (1,3,5-triazinan) group, 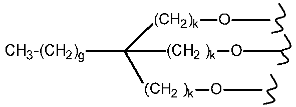 group, 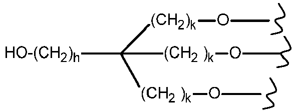 group and 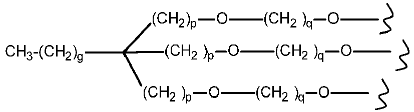
group wherein
g is a natural number selected from 1 to 33,
k is a natural number selected from 1 to 12,
h is a natural number selected from 1 to 15,
p is a natural number selected from 1 to 3 and
q is a natural number selected from 1 to 5,
for n≥4 and E= -CH₂-CH₂-C(O)-
Q is selected from the group of 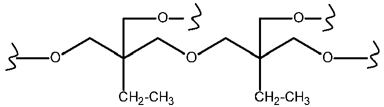 and 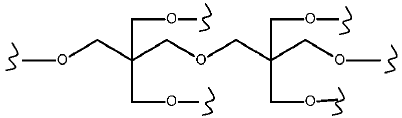
and
with R² selected from the group of H, substituted or unsubstituted C₁-C₄₀ alkyl groups, substituted or unsubstituted C₃-C₄₀ cycloalkyl groups, substituted or unsubstituted saturated or unsaturated heterocyclic groups containing 2 to 20 carbon atoms and one or more heteroatoms selected from the group of N, O, and S as ring members, substituted or unsubstituted C₆-C₄₀ aryl groups, substituted or unsubstituted C₂-C₄₀-alkenyl groups, C₂-C₄₀ alkynyl groups, substituted or unsubstituted C₄-C₄₀ alkylencycloalkyl groups and substituted or unsubstituted C₁-C₄-alkylen groups to be a part of a substituted or unsubstituted saturated or unsaturated heterocyclic group that is built up between R² of the -NR²- group and Y to form 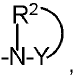
with Y is selected from the group of substituted or unsubstituted C₁-C₄₀ alkyl groups, substituted or unsubstituted saturated or unsaturated C₃-C₄₀ cycloalkyl groups, substituted or unsubstituted C₂-C₄₀ (poly)ether groups, substituted or unsubstituted saturated or unsaturated heterocyclic groups containing 2 to 20 carbon atoms and one or more heteroatoms selected from the group of N, O, and S as ring members, substituted or unsubstituted saturated or unsaturated alkylenheterocyclic groups containing 3 to 20 carbon atoms and one or more heteroatoms selected from the group of N, O, and S as ring members, substituted or unsubstituted C₆-C₄₀ aryl groups, substituted or unsubstituted C₂-C₄₀ alkenyl groups, substituted or unsubstituted C₂-C₄₀ alkynyl groups, substituted or unsubstituted saturated or unsaturated C₃-C₄₀ alkylencycloalkyl groups, substituted or unsubstituted C₇-C₄₀ alkylenaryl groups, substituted or unsubstituted α,β-unsaturated carbonyl groups containing C₃-C₄₀ atoms, substituted or unsubstituted C₂-C₅ keto groups and substituted or unsubstituted C₁-C₄ alkylen group which is part of a substituted or unsubstituted saturated heterocyclic group that is built up between R² of the -NR²- group and Y to form 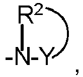
with -A- is one of the following bridging groups: -X¹-Z-X²- or 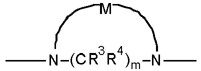
wherein X¹, X² are independently selected from -O-, and-NR²- and
Z is selected from the group of substituted or unsubstituted C₁-C₄₀ alkylen groups, divalently substituted or unsubstituted C₂-C₄₀ (poly)ether groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded substituted or unsubstituted alkylenheterocyclicalkylen groups containing 4 to 20 carbon atoms and one or more heteroatoms selected from the group of N, O, and S as ring members, divalently bonded substituted or unsubstituted C₆-C₄₀ aryl groups, divalently bonded substituted or unsubstituted C₇-C₄₀ cycloalkylalkylencycloalkyl groups, divalently bonded substituted or unsubstituted saturated or unsaturated C₅-C-₄₀ alkylencycloalkylalkylen groups, divalently bonded substituted or unsubstituted C₁₃-C₄₀ arylalkylenaryl groups, divalently bonded substituted or unsubstituted C₈-C₄₀ alkylenarylalkylen groups and divalently bonded substituted or unsubstituted C₁₇-C₃₀ alkylen-bisphenol A - alkylen groups and
R³ and R⁴ are independently selected from the group of H, substituted or unsubstituted C₁-C₄₀ alkyl groups,
m is a natural number selected from the group of 1, 2 or 3 and
M is selected from the group of substituted or unsubstituted C₁-C₃ alkylen groups, divalently bonded -NR⁵-CR³R⁴- groups, divalently bonded -NR⁵-NR⁶- groups, divalently bonded -NR⁵-CR³R⁴-CR³R⁴- groups, divalently bonded-O-CR³R⁴-CR³R⁴- groups, divalently bonded -S-CR³R⁴-CR³R⁴- groups, divalently bonded CR³R⁴-O-CR³R⁴ groups, divalently bonded -CR³R⁴-S-CR³R⁴- groups and divalently bonded -CR³R⁴-NR⁵-CR³R⁴- groups with R⁵ and R⁶ are independently selected from the
group of H, substituted or unsubstituted C₁-C₄₀ alkyl groups, C₆-C₄₀ aryl groups, C₃-C₄₀ cycloalkyl groups and-C(O)CH=CH₂ group and
wherein the alkynyl ether compounds of formula (I) with
n =1, R¹ = H, E = -CH₂-CH₂- and Q= -CN or with
n = 1, R¹ = H, E =-CH₂-CH₂-C(O)- and Q = -O-Y with Y = methyl or ethyl are disclaimed.

2. The alkynyl ether compounds according to claim 1, wherein E = -CH₂-CH₂- , n is selected from the group of 1 and 2 and Q is selected from the group of -CN, with C₁-C₄ alkylgroups or C₁-C₄ alkylhydroxy groups substituted 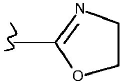 (2-oxazolin) groups, a divalent bonded sulfone (-S(O)₂-), a divalent substituted or unsubstituted 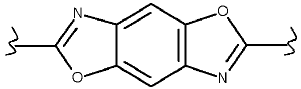 group and a divalent substituted or unsubstituted 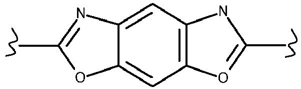 (benzobisoxazol-2,6-diyl) group.

3. The alkynyl ether compounds according to claim 1, wherein E = 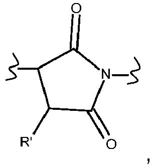 n is selected from the group of 1 and 2 and Q is selected from the group of phenyl, substituted or unsubstituted divalently bonded C₆-C₄₀ aryl groups, divalently bonded substituted or unsubstituted C₁₃-C₄₀ arylalkylenaryl groups, divalently bonded substituted or unsubstituted C₈-C₄₀ alkylenarylalkylen groups.

4. The alkynyl ether compounds according to claim 3, wherein Q is selected from the group of phenyl, 1,4-phenylen, 1,3-phenylen, 4-Methyl-1,3-phenylen, 2,2,4-trimethyl-1,6-hexylen, 1,3-Benzenedimethylene (meta-CH₂-Ph-CH₂-), bis(1,4-phenylen)methane (para -CH₂-Ph-CH₂-) and 3,3'-Diethyl-5,5'-dimethyl-4,4'-diphenylenmethane.

5. The alkynyl ether compounds according to claim 1, wherein E = -CH₂-CH₂-C(O)-, n is se-lected from the group of 1 and 2 and Q is selected from the group of -O-Y and -O-Z-O-.

6. The alkynyl ether compounds according to claim 5, wherein Y is selected from the group of substituted or unsubstituted C₁-C₄₀ alkyl groups, substituted or unsubstituted saturated or unsaturated C₃-C₄₀ cycloalkyl groups, substituted or unsubstituted saturated alkylenheterocyclic groups containing 3 to 20 carbon atoms and one or more heteroatoms selected from the group of N, O, and S as ring members, substituted C₃-C₄₀ unsaturated alkylencycloalkyl groups and
Z which is selected from the group of substituted or unsubstituted C₁-C₄₀ alkylen groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded saturated substituted or unsubstituted C₅-C₄₀ alkylencycloalkylalkylen groups and divalently bonded substituted or unsubstituted C₁₇-C₃₀ alkylen-bisphenol A -alkylen groups.

7. The alkynyl ether compounds according to claim 1, wherein E = -CH₂-CH₂-C(O)-, n =1 or 2 and Q is selected from the group of -NR²-Y, substituted or unsubstituted 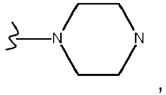 -NR²-Z-NR²-, -NR²-Z-O- and 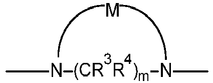 group wherein R¹,R², Y, Z, R³, R⁴, M and m are as defined above.

8. The alkynyl ether compounds according to claim 7, wherein
R² is selected from the group of H and substituted or unsubstituted C₁-C₄₀ alkyl groups,
Y is selected from the group of substituted or unsubstituted C₁-C₄₀ alkyl groups and substituted or unsubstituted C₆-C₄₀ aryl groups, the substituted 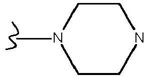 group is a 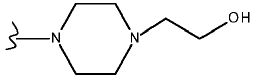 (4-(2-hydroxyethyl)piperazin-1-yl group,
Z is selected from the group of substituted or unsubstituted C₁-C₄₀ alkylen groups, divalently bonded substituted or unsubstituted C₆-C₄₀ aryl groups, divalently bonded substituted or unsubstituted alkylenheterocyclicalkylen groups containing 4 to 20 carbon atoms and one or more heteroatoms selected from the group of N, O, and S as ring members
M is -CH₂-CH₂-,
R³, R⁴ are H and
m is 2.

9. The alkynyl ether compounds according to claim 1, wherein E = -CH₂-CH₂-C(O)-, n is 3 and Q is selected from the group of unsubstituted 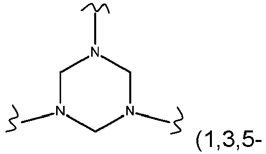 triazinan) group, 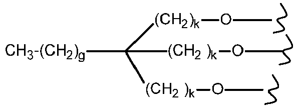 group, 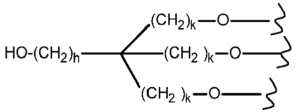 group and 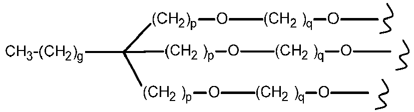 group wherein
g is a natural number selected from 1 to 33,
k is a 1,
h is a natural number selected from 1 to 15,
p is a 1 and
q is a natural number selected from 1 to 5.

10. The alkynyl ether compounds according to claim 1, wherein E = -CH₂-CH₂-C(O)-, n ≥ 4 and Q is selected from the group of 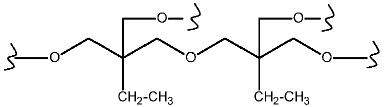 and

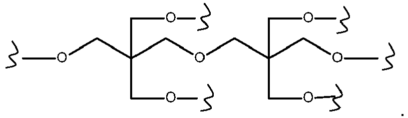

11. A process for the preparation of the alkynyl ether compound of formula (I) according to claim 1 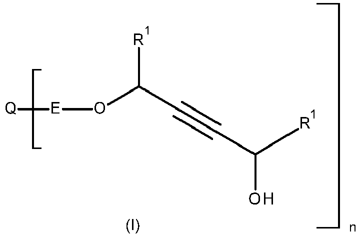 comprising the reaction of an α,β- unsaturated functional compound selected from the group of compounds of formula (II) 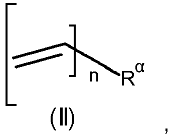 maleinimide-N-Q-group ( 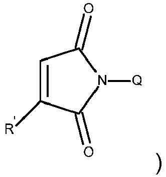 and a maleinimide-N-Q-N-maleinimide group 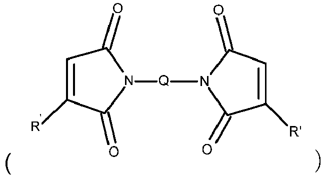 with Q as defined above for the case where n is 1 or 2 and E is 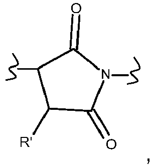 with 1,4,-butynediol and/or 2,5-hexynediol,
wherein
for n = 1, R^{α} is selected from the group of -CN, 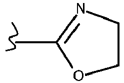 (2-oxazolin), -C(O)-O-Y and -C(O)-NR²-Y,
for n = 2, R^{α} is selected from the group of -C(O)-A-C(O)-, -S(O)₂- and 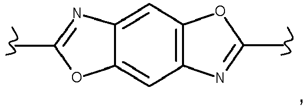 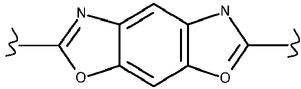 (benzobis oxazol-2,6-diyl),
for n = 3 R^{α} is selected from the group of 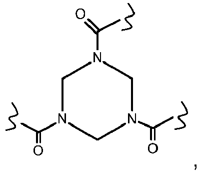 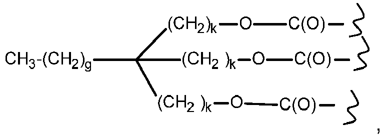 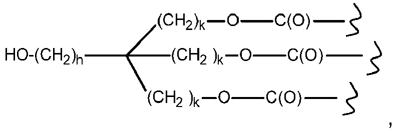 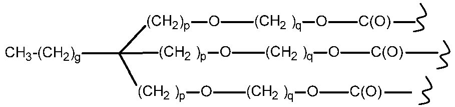 wherein g, k, h, p and q are defined as above,
for n ≥ 4 R^{α} is selected from the group of 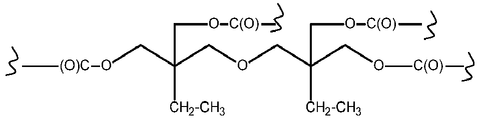 and 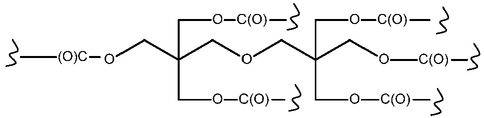 with R', Y, R² and A as defined above.

12. An alternative process for the preparation of the inventive alkynyl ether compound of formula (I) where E = -CH₂-CH₂-C(O)- and Q is selected from the group of -NR²-Y and -X¹-Z-X²- wherein the compounds where X¹ and X² are both -O- are disclaimed 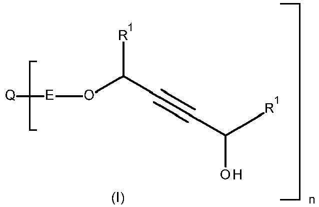 comprising the following steps:
a) reacting an α,β- unsaturated carbonyl compound of formula (IIa) 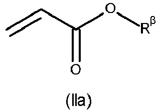
with R^{β} selected from the group of C₃-C₁₀ alkyl groups,
with 1,4,-butynediol and/or 2,5-hexynediol,
wherein the resulting product of step a), which is the ester of formula (III) or a mixture of two E/Z-isomers of the ester of formula (III), 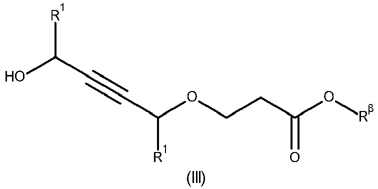
b) reacts in a further step b) with a compound selected from the group of an amine, diamine, triamine and substituted or unsubstituted ethanolamine,
wherein R¹, Y, R², Z, X¹, X² and n are as defined above.

13. The alternative process according to claim 12, wherein the ester of formula (III) as the resulting product of step a) reacts with a compound selected from the group of HNR²-Y, H₂N-Z-NH₂, 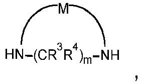 N-substituted piperazine, substituted or unsubstituted 1,3,5-triazinane, H₂N-Z-OH, wherein Y, R², Z, M, R³, R⁴ and m are as defined above.

14. The process according to any of claims 11 to 13, wherein the molar ratio between 1,4-butynediol and/or 2,5-hexynediol and α,β- unsaturated functional compound of formula (II) or α,β- unsaturated carbonyl compound of formula (Ila) is in the range from 1:1 to 10:1.

15. The process according to any of claims 11 to 14, wherein the temperature during the reaction of the α,β- unsaturated functional compound of formula (II) or the α,β-unsaturated carbonyl compound of formula (IIa) with 1,4-butynediol and/or 2,5-hexynediol will be in the range from 25°C to 120°C, at normal pressure and a catalyst is used.

16. The process according to any of claims 11 to 15, wherein an alkaline catalyst is used during the reaction of the α,β- unsaturated functional compound of formula (II) or α,β-unsaturated carbonyl compound of formula (IIa) with 1,4-butynediol and/or 2,5-hexynediol.

17. The process according to any of the claims 11 to 14, wherein no solvent is used during the reaction of the α,β- unsaturated functional compound of formula (II) or α,β-unsaturated carbonyl compound of formula (IIa) with 1,4-butynediol and/or 2,5-hexynediol.

18. The process according to claim 15, wherein the molar ratio between 1,4-butynediol and/or 2,5-hexynediol and α,β- unsaturated functional compound of formula (II) or α,β-unsaturated carbonyl compound of formula (IIa) is in the range from 3:1 to 10:1.

19. The process according to any of claims 11 to 13, wherein a polar organic solvent is used during the reaction of the α,β- unsaturated functional compound of formula (II) or α,β- unsaturated carbonyl compound of formula (IIa) with 1,4-butynediol and/or 2,5-hexynediol.

20. The process according to any of the claims 11 to 13, wherein an aqueous solvent is used during the reaction of the α,β- unsaturated functional compound of formula (II) or α,β- unsaturated carbonyl compound of formula (IIa) with 1,4-butynediol and/or 2,5-hexynediol.

21. The process according to claim 18, wherein the aqueous solvent is water only and the molar ration between 1,4-butyndiol and/or 2,5-hexyndiol and α,β- unsaturated functional compound of formula (II) or α,β- unsaturated carbonyl compound of formula (Ila) is in the range from 1:1 to 3:1.

22. The process according to any of the claims 11 to 12, wherein the α,β- unsaturated functional compound of formula (II) or α,β- unsaturated carbonyl compound of formula (IIa) is dissolved in a polar organic solvent and 1,4-butyndiol and/or 2,5-hexyndiol are dissolved in water.

23. The process according to any of the claims 11 to 12, wherein the α,β- unsaturated functional compound of formula (II) or α,β- unsaturated carbonyl compound of formula (IIa) is either dissolved in an organic solvent or no solvent.

24. Aqueous mixture obtained by the process according to claim 21.

25. Mixture obtained by the process according to claim 11, comprising at least two alkynyl ether compounds of formula (I), wherein in one alkynyl ether compound of formula (I) R¹ is H and in the other alkynyl ether compound of formula (I) R¹ is methyl.

26. Use of the alkynyl ether compounds according to any of claims 1 to 10 for the preparation of cyclic carbonates with exocyclic vinylidene groups of formula (IV) 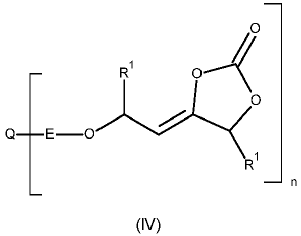 wherein R¹, E, Q and n are as defined above.

27. Use of the alkynyl ether compounds of formula (I) according to any of claims 1 to 10 as corrosion protection agent.

28. Use of the mixture according to claim 22 for the preparation of a mixture of cyclic carbonates with exocyclic vinylidene groups of formula (IV) where in some cyclic carbonates with exocyclic vinylidene groups R¹ is H and in the others R¹ is methyl.

29. Use of the aqueous mixture according to claim 21 as corrosion protection agent.
